(19) Europäisches Patentamt
European Patent Office
Office européen des brevets

(11) **EP 4 513 425 A1**

(12) **EUROPEAN PATENT APPLICATION**

(43) Date of publication:
**26.02.2025 Bulletin 2025/09**

(21) Application number: **24196019.4**

(22) Date of filing: **22.08.2024**

(51) International Patent Classification (IPC):
**G06T 5/60** (2024.01)  **G06T 7/00** (2017.01)

(52) Cooperative Patent Classification (CPC):
**G06T 5/60; G06T 7/0012;** G06T 2207/10081;
G06T 2207/10088; G06T 2207/20081;
G06T 2207/20084

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.08.2023 GB 202312835**

(71) Applicant: **Elekta Limited
Montreal, Québec H3A 2J5 (CA)**

(72) Inventors:
• **Beriault, Silvain**
  H3A 2J5 Montreal (CA)
• **Savard, Laurence**
  H3A 2J5 Montreal (CA)
• **Alain-Beaudoin, Alexandra**
  H3A 2J5 Montreal (CA)

(74) Representative: **Hamer, Thomas Daniel
Kilburn & Strode LLP
Lacon London
84 Theobalds Road
London WC1X 8NL (GB)**

(54) **GENERATING SYNTHETIC IMAGE**

(57) Disclosed herein is a method for generating one or more synthetic electron density, sED, images. The method comprises obtaining a first image of a first imaging modality, the first image depicting an anatomical region of a subject. The method also comprises generating, using a trained machine learning model and the first image, a sED image depicting the anatomical region, wherein the trained machine learning model has been trained using a set of training images comprising a first subset of training images of the first imaging modality, and a second subset of training images in which each training image comprises electron density, ED, information.

Fig. 1

EP 4 513 425 A1

**Description**

This disclosure relates to generating synthetic images, and in particular to generating synthetic images based on a first image of a first imaging modality, for example to generate synthetic CT or CBCT images based on a magnetic resonance image. This disclosure also relates to a new training strategy to generate synthetic Electron Density (ED) based on input images such as MRI images in an end-to-end fashion.

Background

Radiotherapy can be described as the use of ionising radiation, such as X-rays, to treat a human or animal body. Radiotherapy is commonly used to treat cancer, for example to treat tumours within the body of a patient or subject. In such treatments, ionising radiation is used to irradiate, and thus destroy or damage, cells which form part of the tumour.

Medical imaging has become increasingly important in the diagnosis and treatment of oncological patients, particularly in radiotherapy (RT). Before radiotherapy treatment, a treatment plan is created to determine how and where the radiation should be applied. Typically, such a treatment plan is created with the assistance of medical imaging technology. For example, a CT scan may be taken of the patient in order to produce a three-dimensional image of the area to be treated. The three-dimensional image allows the treatment planner to observe and analyse the target region and identify surrounding tissues. This initial CT-image, on which the treatment plan is based, may be referred to as a reference image.

Different structures within the body of the patient, for example bone, lung, muscle, etc., will attenuate and absorb radiation to differing degrees based on their respective densities. In other words, different tissues within the human body have different radiodensities, and hence attenuate and/or absorb radiation to differing degrees. Bone is an example of a particularly radiodense or radiopaque tissue. In contrast, soft tissue, for example lung tissue, is radiolucent. The radiodensity of various tissues can be quantified using the Hounsfield scale, using Hounsfield Units (HU) in a manner which is known to the skilled person. The Hounsfield scale is a measure of the degree of attenuation of the X-Rays as they pass through the patient's body.

However, while CT images use Hounsfield Units, in order to plan radiotherapy treatment it is necessary to obtain information regarding the electron density information; not only for the target region, such as a tumour, but also surrounding tissues and any regions of the body through which the radiation treatment beam will pass. CT-values are related to electron density, and it is possible to convert Hounsfield Units (HU) to electron density values using calibration curves, for example. Calibration curves can be created using calibration phantoms comprised of different materials with known electron density values. The resulting calibration curve is distinctive to the particular imaging scanner on which the calibration is performed.

Radiotherapy treatment is typically split into several treatment sessions, called "fractions". The patient's anatomy can change between fractions, and it is important to ensure the patient's geometry on the day of treatment is "aligned" with the geometry of their anatomy shown in the reference image. To assist with this, a "daily" image can be taken just before treatment is to commence. Such "daily" images could be CT or magnetic resonance (MR) images, for example. This allows clinicians to position the patient accurately on the table. In addition to allowing accurate positioning, it is desirable to use the daily image as the basis for adjustments or optimisations to the treatment plan itself, for example to account for changes in the patient's anatomy.

Obtaining daily MR images does not involve exposing a patient to ionising radiation, and hence does not provide any dose to the patient. MR images provide good soft tissue contrast, allowing a treatment planner to better distinguish between, for example, tumour tissue and prostate tissue. The disadvantage of MR scanning is that it does not indicate the attenuation properties of tissues within the subject, or provide the electron density for the patient's tissues which is required to create a radiation treatment plan. Magnetic resonance linear accelerator (MR-linac) devices allow clinicians to take daily MR images just before radiotherapy is to be delivered.

When taking daily MR images, e.g. with a combined magnetic resonance linear accelerator (MR-linac) device, if any type of treatment plan adjustment or "re-optimisation" is desired then it is necessary to somehow generate the electron density information. One current workflow for MR-linacs involves taking the reference image, segmenting it to produce contoured organs in the reference image and assigning average ED values to the contoured organs. This is a very coarse approximation of ED values. Alternatively, the reference CT image may be registered or deformed to the daily MR image. The resulting deformed image can then be used as the basis of plan updates and re-optimisations. However, this type of deformable registration can produce inaccuracies, particularly when registering images acquired using different modalities in this way (e.g. CT to MR). This approach works well but requires the registration itself to be highly accurate, otherwise the treatment re-optimisations may be sub-optimal.

In addition, people have speculated that it may be possible to use a trained machine learning model in the radiotherapy workflow. For example it is known to train a neural network for supervised image-to-image translation using paired treatment images, e.g. pairs of MR and CT images, to generate a synthetic or 'pseudo-' CT image based on an MR image. Such a trained neural network could generate a synthetic CT image from the daily MRI. By generating an image directly with machine learning , the registration between the reference CT and the synthetic CT image can be made more accurate. Again, this approach may work well, but the present inventors have realised that further efficiencies can be achieved in the radiotherapy workflow.

The present invention seeks to address these and other disadvantages encountered in the prior art.

Summary

An invention is set out in the independent claims. Optional features are set out in the dependent claims.

Figures

Specific embodiments are now described, by way of example only, with reference to the drawings, in which:

Figure 1 depicts a radiotherapy device or apparatus according to the present disclosure;

Figure 2 depicts a method of generating a synthetic image according to the present disclosure;

Figure 3 depicts a method of training a model in accordance with the present disclosure;

Figure 4a depicts a GAN model in accordance with the present disclosure;

Figure 4b depicts an architecture for a generator in accordance with the present disclosure;

Figures 5a-c depict the results of a multiple channel approach in accordance with the present disclosure, for example to eliminate the 'staircase effect';

Figures 6a-b show representative examples of MR-to-sCT translation;

Figure 7 is a graph showing different CT-ED calibration curves for different scanners;

Figure 8 depicts a method of training a machine learning model in accordance with the present disclosure;

Figure 9 depicts a method of generating a synthetic ED image, and a radiotherapy workflow, both according to the present disclosure;

Figure 10 shows a high-level overview of a CycleGAN;

Figure 11 depicts a system according to the present disclosure;

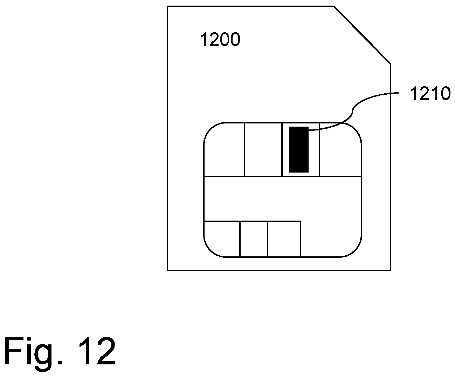

Figure 12 depicts a storage medium according to the present disclosure.

Detailed Description

In overview, and without limitation, the present application relates broadly to two aspects. These aspects and their respective features can be used together, or separately.

A CT image not only provides information about the patient geometry by providing a three-dimensional image of the patient, but also provides information regarding the radiodensities of the different tissues and structure within the patient's body. A CT scan typically produces a three-dimensional image comprised of voxels, where each voxel is assigned a CT value. Each voxel is associated with a particular location within the patient's body, and the CT-values of the voxels together de-scribe the radiodensity of tissues within the patient's body. The CT-values are indicative of the attenuation properties of tissues within the patient's body.

However, a CT scan involves irradiating the patient from a number of angles in order to produce the three-dimensional image. Therefore, a disadvantage of CT scanning is that it adds a radiation dose to the patient even before treatment has started. Also, while CT scans provide the necessary information regarding electron densities for radiation treatment planning, CT scans can also provide poor soft tissue contrast. This makes it difficult for a treatment planner to distinguish between certain kinds of soft tissue. For example, it is very difficult to see a tumour in a prostate CT scan, because the tumour and the prostate have very similar densities and attenuation properties and thus look similar, or even identical, in a CT image.

It is already possible to combine MR and CT data to facilitate the treatment planning process. It is known that MR images and CT images may be obtained independently and then aligned with each other, for example by aligning various discernible locations of features of interest in both images. Such alignment, or fusing, may involve either rigid or deformable adjustment of the MR image to align with the CT image to produce a pair of co-aligned images. However, such alignment, or registration, still re-quires a CT image to be taken, thereby providing a radiation dose to a patient, and this approach takes time, increases workload, and sometimes has sub-optimal accuracy.

A certain degree of progress in the field has also been made by creating so-called 'pseudo-CT', or synthetic CT (sCT) images. These are images similar to those obtained from a CT scan, i.e. which comprise tissue radiodensity information in Hounsfield Units in a manner similar to a 'real' CT image, but which are derived primarily or entirely from MR data such as an MR image of the patient. Recently, MRI-only based radiotherapy has been proposed to simplify and speed up the radiotherapy workflow, thereby decreasing patients' exposure to radiation. MRI-only RT may also reduce overall treatment costs and workload, and eliminate residual registration errors when using both imaging modalities. Additionally, the development of MRI-only techniques can be beneficial for MRI-guided RT such as can be accomplished using an MR-linac, for example. However, an issue with known approaches, and an obstacle to introducing MRI-only RT, is the lack of accurate tissue attenuation and electron density information required for accurate dose calculations.

Therefore, according to a first aspect, the application discloses methods of generating a synthetic CT image which are particularly beneficial for MR-guided external beam radiotherapy (MRgRT). This application area can strongly benefit from AI-powered image-to-image translation, e.g. for generating synthetic CT (sCT) contrast from MR images. This process can be referred to as MR-to-sCT. MR-to-sCT is an enabler for MR-only radiotherapy workflows. Such workflows can avoid the use of ionizing radiation imaging, while providing radiation-oncologists (RO) with enhanced soft-tissue visualization (with MRI) alongside co-registered sCT contrast with proper Hounsfield Units (HU) to infer electron density (ED) values for RT dose calculation. Moreover, MR-to-sCT can significantly enhance current online plan adaptation techniques in MRgRT. For example, current online adaptive workflows with Unity MR-Linac (Elekta AB, Stockholm, Sweden) require manual or semiautomatic contouring of different structures on the daily MRI, including all bony anatomy. Bulk electron density values are then assigned to each contoured structure as part of the plan adaptation process. MR-to-sCT could provide more realistic HU and ED values, while significantly reducing the burden of re-contouring all bony anatomy on each daily MRI.

According to a second aspect, the application also discloses a computer-implemented method for generating one or more synthetic electron density (sED) images, a method for training a machine learning model for use in generating one or more sED images, and a new radiotherapy workflow which makes use of the sED images to optimise or 're-optimise' a treatment plan.

In this second aspect, in a training stage, a machine learning model is trained using a set of training images comprising a first subset of training images of a first imaging modality, for example MR images, and a second subset of training images in which each training image comprises electron density information. By training a machine learning model using training images which comprise electron density information according to the methodologies described herein, the resulting trained model is able to generate synthetic electron density (sED) images which comprise synthetic electron density information. This information is required, for example, when re-optimising a radiotherapy treatment plan on the day of treatment.

In an inference stage, the trained machine learning model can be used to convert a daily MR, or a daily image of another modality, to a sED image for use in re-optimisation processes (for example fluence or segment re-optimisation). The method comprises receiving a first image of a first imaging modality, for example an MR image. This first image depicts an anatomical region of a subject, such as the tumour and surrounding tissues for a patient undergoing radiotherapy. Using the trained machine learning model and the first image, a sED image can then be generated which depicts the anatomical region.

When plan re-optimisation is desired, prior radiotherapy workflows have made use of deformable MR-CT registration techniques. While this approach, when implemented properly, is safe and accurate, it is desirable to improve accuracy and efficacy of treatment still further. The approach can introduce small inaccuracies, particularly when the patient's anatomy has changed inter-fraction. The disclosed method and associated improved radiotherapy workflow does not involve deformable MR-CT registration, and therefore does not suffer from any associated inaccuracies.

Some other approaches have sought to segment the daily MR image, and then assign the contoured tissue within the segmented MR image bulk electron density values based on the contoured tissue. For example, tissue identified as being rectal tissue may be assigned a particular ED value, and tissue identified as being sacral bone issue may be assigned a different ED value. This bulk assignment approach can also work well, but requires the anatomy to be fully contoured. Still other approaches have sought to generate a synthetic CT image using the daily MR image, but again this necessitates additional steps in order to produce a suitable image for plan re-optimisation which includes the electron density information. This introduces a delay and may need involvement from a clinician. By training a machine learning model using training images which comprise electron density information according to the methodologies described herein, the trained model is able to generate a sED and the need for additional workflow steps is avoided. The disclosed method is therefore more quick, efficient, and makes better use of clinician's time. In turn, treatment throughput is improved.

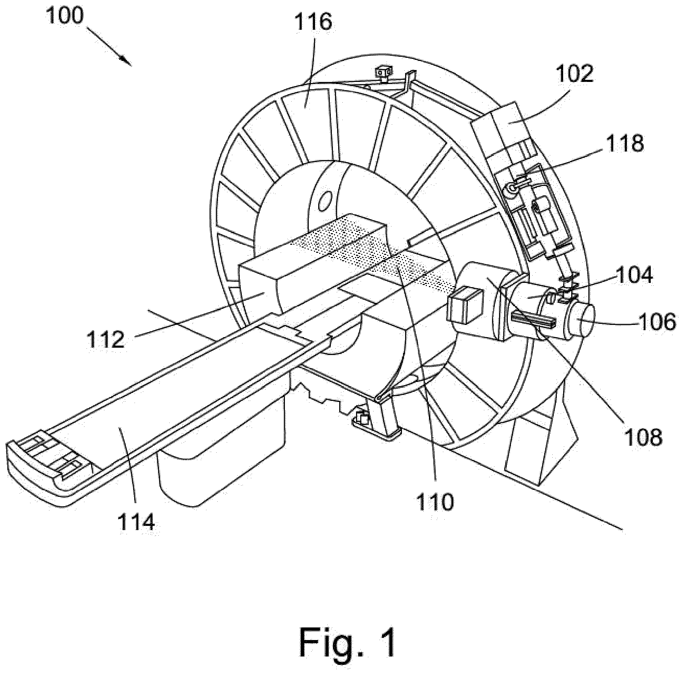

Figure 1 depicts an MR-linac radiotherapy device suitable for delivering, and configured to deliver, a beam of radiation to a patient during radiotherapy treatment. The device and its constituent components will be described generally for the purpose of providing useful accompanying information for the present invention. The device depicted in figure 1 is in accordance with the present disclosure and is suitable for use with the disclosed systems and apparatuses. While the device in figure 1 is an MR-linac, the generation of synthetic images, in particular sCT and/or sED images, may enable workflows on any radiotherapy device, even one without an MR imager, for example a traditional linac device.

The device 100 comprises both MR imaging apparatus 112 and radiotherapy (RT) apparatus which may comprise a linac device. The MR imaging apparatus 112 is shown in cross-section in the diagram. In operation, the MR scanner produces MR images of the patient, and the linac device produces and shapes a beam of radiation and directs it toward a target region within a patient's body in accordance with a radiotherapy treatment plan.

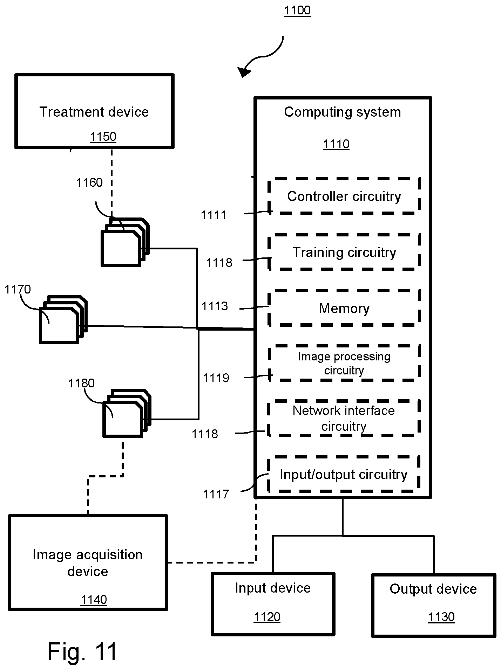

The MR-linac device depicted in figure 1 comprises a source of radiofrequency waves 102, a waveguide 104, a source of electrons 106, a source of radiation 106, a collimator 108 such as a multi-leaf collimator configured to collimate and shape the beam, MR imaging apparatus 112, and a patient support surface 114. In use, the device may also comprise a housing (not shown) which, together with the ring-shaped gantry, defines a bore. The moveable support surface 114 can be used to move a patient, or other subject, into the bore when an MR scan and/or when radiotherapy is to commence. The MR imaging apparatus 112, RT apparatus, and a subject support surface actuator are communicatively coupled to a controller or processor. The controller is also communicatively coupled to a memory device comprising computer-executable instructions which may be executed by the controller or processor, e.g. in the manner depicted in figure 11.

The radiation source may comprise a beam generation system. For a linac, the beam generation system may comprise a source of RF energy 102, an electron gun 106, and a waveguide 104. The radiation source is attached to the rotatable gantry 116 so as to rotate with the gantry 116. In this way, the radiation source is rotatable around the patient so that the treatment beam 110 can be applied from different angles around the gantry 116. In a preferred implementation, the gantry is continuously rotatable. In other words, the gantry can be rotated by 360 degrees around the patient, and in fact can continue to be rotated past 360 degrees. The gantry may be ring-shaped. In other words, the gantry may be a ring-gantry.

The source 102 of radiofrequency waves, such as a magnetron, is configured to produce radiofrequency waves. The source 102 of radiofrequency waves is coupled to the waveguide 104 via circulator 118, and is configured to pulse radiofrequency waves into the waveguide 104. The source of radiation is configured to direct a beam 110 of therapeutic radiation toward a patient positioned on the patient support surface 114. The source of radiation may comprise a heavy metal target toward which the high energy electrons exiting the waveguide are directed. When the electrons strike the target, X-rays are produced in a variety of directions. A primary collimator may block X-rays travelling in certain directions and pass only forward travelling X-rays to produce a treatment beam 110. The X-rays may be filtered and may pass through one or more ion chambers for dose measuring. The beam can be shaped in various ways by beam-shaping apparatus, for example by using a multi-leaf collimator 108, before it passes into the patient as part of radiotherapy treatment.

The subject or patient support surface 114 is configured to move between a first position substantially outside the bore, and a second position substantially inside the bore. In the first position, a patient or subject can mount the patient support surface. The support surface 114, and patient, can then be moved inside the bore, to the second position, in order for the patient to be imaged by the MR imaging apparatus 112 and/or imaged or treated using the RT apparatus

The radiotherapy apparatus / device depicted in figure 1 also comprises MR imaging apparatus 112. The MR imaging apparatus 112 is configured to obtain images of a subject positioned, i.e. located, on the subject support surface 114. The MR imaging apparatus 112 may also be referred to as the MR imager. The MR imaging apparatus 112 may be a conventional MR imaging apparatus operating in a known manner to obtain MR data, for example MR images. The skilled person will appreciate that such a MR imaging apparatus 112 may comprise a primary magnet, one or more gradient coils, one or more receive coils, and an RF pulse applicator. The operation of the MR imaging apparatus is controlled by the controller.

The controller is a computer, processor, or other processing apparatus. The radiotherapy device 100 may form part of a radiotherapy system along with a controller / processor in the manner shown and described with respect to figure 11.

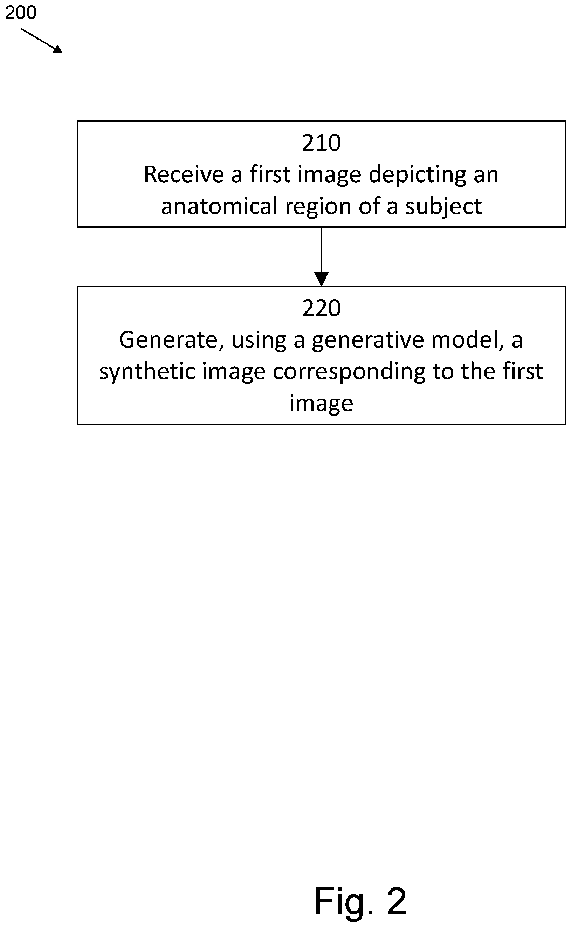

Figure 2 is a flowchart depicting a method 200 according to the present disclosure at a high level. The method is for generating a synthetic image.

At block 210, a first image is received. The first image is of a first imaging modality, and e.g. may have been acquired using imaging apparatus of a first imaging modality. The first imaging modality may be MR, such that the first image is an MR image. The first image depicts an anatomical region of a subject. For example, the first image may depict the pelvis or brain of a patient. At the time of imaging, the patient may be positioned on the subject / patient support surface of a radiotherapy machine such as an MR-linac. Alternatively, at block 210, first imaging data may be acquired, where the data is representative of the first image.

At block 220, a synthetic image is generated using a generative model. The synthetic image corresponds to the first image, and may for example depict the same anatomical region of the patient, or at least appear to. The synthetic image corresponds to a second imaging modality. The synthetic image may take the form of a second imaging modality image. The synthetic image may represent, or be similar to an image of the second imaging modality, without having been acquired using imaging apparatus of the second imaging modality. The second imaging modality may be any of computed tomography (CT) and cone-beam computed tomography (CBCT). For example, the image may be an sCT image.

In an implementation, generating the synthetic image may comprise generating synthetic imaging data using the generative model, where the synthetic imaging data is representative of a region of the input (first) image. For example, the input image may be subdivided into a plurality of image samples, each sample representing a region of the input image, and these samples can be presented as input to the generative model in order to generate corresponding regions of the synthetic image. This process can be repeated multiple times in order to generate synthetic imaging data representing multiple different, overlapping regions of the input image. The method may then comprise aggregating the results. For example, where the input is a 3D image and the synthetic imaging data comprises synthetic CT values generated for voxels of the input image, aggregating the results may comprise calculating an average synthetic CT value from each of the synthetic CT values generated by the model.

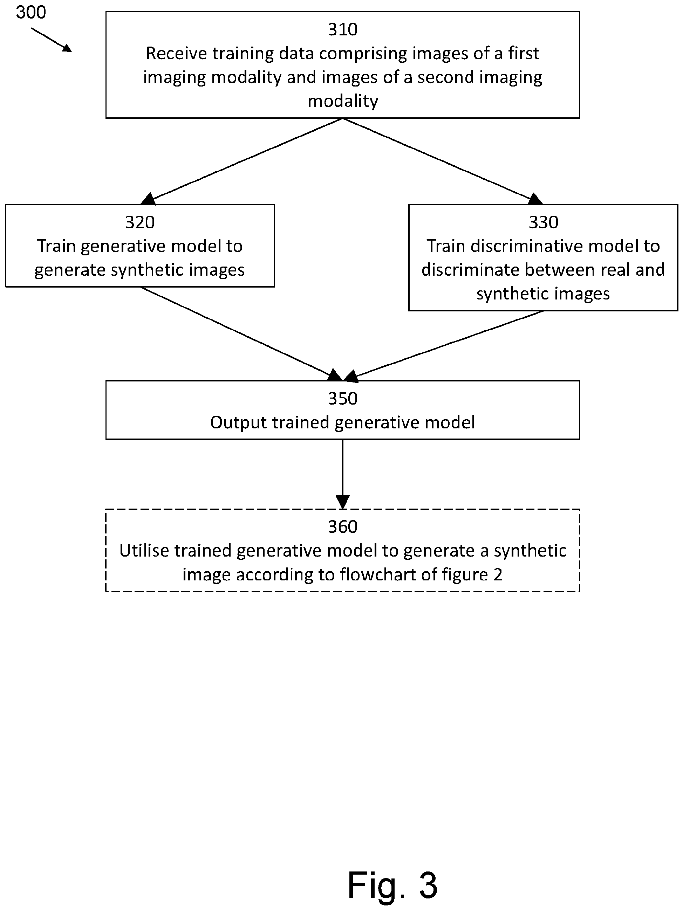

Figure 3 depicts an example training method 300 for training the generative model. As shown in figure 3, the generative model may be trained via a generative adversarial network (GAN). The GAN may comprise a first generator convolutional network and a first discriminator convolutional network.

At block 310, a training set of images is received, which comprises images of a first imaging modality and images of a second imaging modality. These images may be real images. The images may comprise real MR images (either T1 or T2 weighted, or a mixture of both) and real CT images. These images may be paired, such that each pair of images comprises a real MR and real CT image, the images being registered to one another. The CT and MR images of a particular pair of images depict the same patient, and in particular depict the same anatomical region of that patient to enable the images to be registered. However, different image pairs may depict different patients. Suitable training data is discussed in greater detail below.

At blocks 320 and 330, generative and discriminative models are trained in a GAN. At block 320, a generative model, such as a generator convolutional network, is trained to generate synthetic images based on input training images of the first imaging modality, for example based on MR images. These synthetic images correspond to the second imaging modality. The generator network's 'goal' is to create new data instances in the form of synthetic CT images that resemble the real CT images in the training dataset. At block 330, a discriminative model such as a discriminator convolutional network is configured to discriminate between the synthesized images generated by the generative model, and the actual images comprised within the training data. The discriminator network's 'goal' is to distinguish between real CT images in the training dataset and fake, synthetic images generated by the generator. In this way, the output of the generative and discriminative networks can be used to train each other in an adversarial manner, as is understood by the skilled person.

Block 320 may comprise providing, as an input to the generative model, images or imaging data based on the images of the first imaging modality, and receiving estimated synthetic imaging data as an output from the generative model. It can then be determined, using the discriminative model in the GAN, at least one difference between the resulting estimated synthetic imaging data and real imaging data based on the images of the second imaging modality. One or more parameters of the generative model can then be updated based on the determined at least one difference. This process can be performed iteratively until a stopping criterion is reached, for example a stopping criterion relating to the number of passes through the iterative process, or relating to the determined "at least one difference", e.g. a similarity metric between synthetic and real imaging data is reached.

At block 350, a trained generative model is output following the adversarial training process, and may be used to generate sCT or other synthetic images in the manner depicted in figure 2.

The skilled person will understand that, where reference is made to an "image" herein, particularly with reference to the input or output of a machine learning model such as a generative or discriminative model, this should be considered to encompass the use of parts, regions or patches of an image. It should be understood that where reference is made to an "image" herein, this encompasses the use of "imaging data" such as images samples and patches, which incorporate pixel and voxel values and associated values such as CT scores associated with pixels and/or voxels. Reference is made to the methods of both figures 2 and 3. The first image may be a three-dimensional (3D) image comprising a plurality of voxels. As is known to the skilled person, a 3D image can be subdivided in a number of ways, for example into a plurality of 2D image slices arranged perpendicular to any of the three image axes. These image axes may define image planes. The 3D image may therefore be divided into a first plurality of 2D image slices arranged perpendicular to a first image axis, and into two different pluralities of 2D image slices, each arranged perpendicular to a second and third image axis respectively.

As described in more detail below with respect to the disclosed case study implementing the disclosed method, methods of the present disclosure may involve using a multi-channel "2.5D" approach in order to improve the translation results in comparison to applying a 2D machine learning model independently to each 2D image slice. Using this approach, generating a synthetic image may comprise providing, as an input to the generative model, a plurality of consecutive 2D image patches. Each 2D image patch depicts a 2D region in the first image. When considered together, the plurality of consecutive 2D image patches may be referred to as an image sample. The consecutive 2D image patches are arranged perpendicular to a first image axis. The sample size may be, for example, 5 consecutive 2D image patches, where each 2D patch comprises 192x192 voxels. This image sample can be described as a stack of 5 2D image patches.

Following this approach, when the model is trained e.g. according to the method of figure 3, the method may comprise randomly sampling groups of 5 consecutive slices of imaging data and supplying them to the generator and/or the discriminator. At inference time, for example when an sCT is to be generated according to the method of figure 2, the first (input) image is subdivided into patches and/or samples that overlap both in-plane and thru-plane.

In this implementation, it is possible to consider three example image samples, or three 'stacks' of 2D image patches. Each stack of 2D image patches comprises a plurality of consecutive, adjacent 2D image patches. The first and second stack of 2D image patches are aligned along the first image axis, and the third stack of 2D image patches is aligned along a second (different) image axis. These axes may be perpendicular to one another. The first stack and the second stack overlap, such that a first subset of voxels is comprised within both the first stack and the second stack of 2D image patches. Similarly, the first stack and the third stack overlap, such that a second subset of voxels is comprised within both the first stack and the third stack of 2D image patches.

When each of these stacks is passed through the generator network to generate first, second, and third synthetic image data, the voxels which are common to multiple 'stacks' of image patches will each have multiple values associated therewith. The 'final' values for each voxel in the synthetic image are an aggregation of each value generated in the aggregation stage. For example, the final voxel value may be an average value. In a particular implementation, the aggregation involves performing a weighted average, where the weight for any voxel value of a particular image patch is based on its proximity to the centre of the image patch. This means that a central pixel / voxel in the central image slice of an image sample will have a high weighting, and a pixel / voxel at the edge of an image sample will have a low weighting.

In another example implementation, a stack of axial slices is supplied to the network, e.g. 5 slices. At training time, a stack of 5 consecutive slices at a random position within the 3D image is sent to the network. At inference time the following is performed:

A. Send slices 0-4 (axial)
B. Send slices 1-5 (axial)
C. Send slices 2-6 (axial)
D. Send slices 3-7 (axial)
E. Send slices 4-8 (axial)
F. Send slices 5-9 (axial)

And so on. In other words, stacks of consecutive slices are supplied to the network, and the stacks are comprised of slices that increment along the entire image by adding a single slice from one 'end' of the stack and removing a slice form the opposite 'end'. Then, the synthetic CT is computed as follows:

Slice 0 (axial) = A[0]
Slice 1 (axial) = mean (A[1], B[0])
Slice 2 (axial) = mean (A[2], B[1], C[0])
Slice 3 (axial) = mean (A[3], B[2], C[1], D[0])
Slice 4 (axial) = mean (A[4], B[3], C[2], D[1], E[0])
Slice 5 (axial) = mean (B[4], C[3], D[2], E[1]. F[0])

And so on. The results are significantly smoother vertically compared to the prior methods.

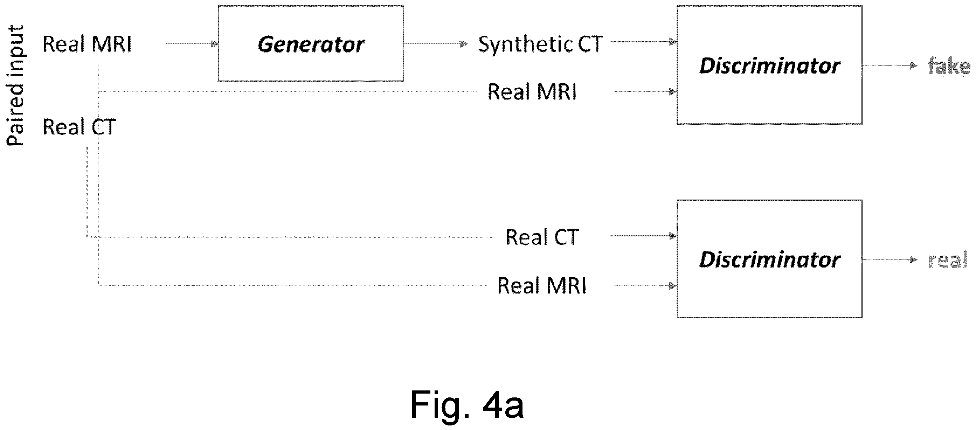

Figure 4a depicts an example machine learning model that comprises a generative model in accordance with the present disclosure. The model can be trained using training data, for example training data as discussed above with respect to block 310 of figure 3. The training data comprises a series of paired input images, for example co-registered MRI and CT images of the same patient. The generator, which may also be described as a generator network, takes as input one or multiple (consecutive) real MRI slices and generates as output one or multiple (consecutive) synthetic CT slices. For example, a standard residual U-Net may be used for the generator network.

The discriminator, which may also be describes as a discriminator network, takes as input one or multiple real MRI slices and one or multiple real or synthetic CT slices. The goal of the discriminator is to guess the supplied CT images as either real or fake (synthetic). Once the network is trained, the generator network is used for inference.

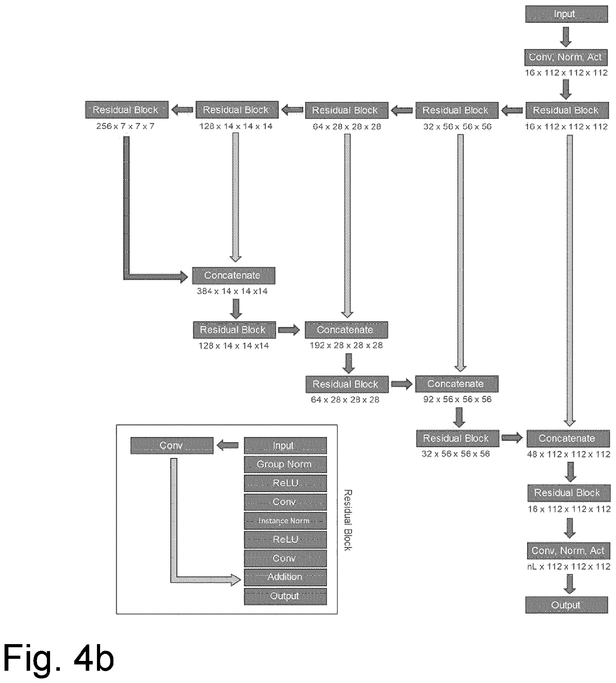

Figure 4b depicts a generator network which may be employed in certain implementations of the present disclosure. The depicted generator is a ResUnet generator. The skilled person will understand that the filter sizes are variable; and in a preferred implementation the filter sizes are not in line with those depicted in figure 4b but as are as follows:

- Input layer: and first layer 64x5x152x152 instead of 16x112x112x112.
- Second layer: 128x5x76x76
- Third layer: 256x5x38x38
- Fourth layer: 512x5x19x19

The discriminator network is not shown, but may be a patch discriminator network and implemented similarly to the generator's encoder portion.

A specific case study and implementation of the methods and machine learning models described herein will now be described for the purposes of aiding understanding.

In the specific implementation, the method is for MR-to-sCT image translation using paired training data. The method is based on the Pix2Pix conditional GAN architecture. A multi-channel (2.5D) approach is used to improve translation results thru-plane in comparison to applying a 2D model independently on each slice, while keeping inference time small in comparison to a full 3D approach. Separate models were trained for both brain (T1-weighted) and pelvis (T1- and T2-weighted) using already paired data. According to this approach, models have been validated using 60 validation subjects. Image similarity metrics obtained during the validation phase are: mean absolute error (MAE) of $64.27 \pm 14.15$, peak signal-to-noise ratio (PSNR) of $28.64 \pm 1.77$, structure similarity index (SSIM) of $0.872 \pm 0.032$.

In terms of prior art for this case study, one method for generating realistic sCT is to use deformable image registration of the patient planning CT with the daily MRI. However, deformable registration across two different modalities is limited in accuracy, especially for highly elastic structures like the bladder.

AI methods based on Generative Adversarial Networks (GANs) can be used for image-to-image translation in medical imaging applications. For example, conditional GANs have been used to train models with paired and accurately registered training images. Alternatively, CycleGANs can be used when training data is unpaired. As such, they can be trained using unmatched training samples that are only available in one modality (MR or CT).

This case study describes a method to enable MR-to-sCT translation, with image data depicting both pelvis and brain tumor sites. All training samples used for this case study are paired, and for this reason a conditional GAN architecture has been adopted. Section 2 below provides implementation details about the method. Section 3 describes strategies for hyperparameter optimization and summarizes the results obtained during a validation phase. Section 4 discusses specific design decisions.

***First Case Study***

**Method**

**1.1 Training Data**

Fully anonymized data was collected from 3 different sites: Radboud University Medical Center, University Medical Center Utrecht, University Medical Center Groningen. The case study described herein consists of 3 phases: training phase, validation phase and test phase. For the training phase, a set of 180 brain MRI/CT pairs and 180 pelvis MRI/CT pairs was used. The MR and CT data were rigidly aligned. An evaluation mask was also used. All brain MRIs were T1-weighted. 120 pelvis MRIs were T1-weighted. The remaining 60 pelvis MRIs were T2-weighted. For the validation phase, a set of 30 brain MRI images and 30 pelvis MRI images was provided to the participants. The corresponding CT images were not used.

More information regarding suitable dataset(s) can be found in the following two papers:

- Thummerer A, van der Bijl E, Galapon Jr A, Verhoeff JJ, Langendijk JA, Both S, van den Berg CAT, Maspero M. 2023. SynthRAD2023 Grand Challenge dataset: Generating synthetic CT for radiotherapy. Medical Physics, 50(7), 4664-4674. https://doi.org/10.1002/mp.16529
- Huijben E, Terpstra ML, Galapon Jr A, Pai S, Thummerer A, Koopmans P, ... & Maspero M. 2024. Generating Synthetic Computed Tomography for Radiotherapy: SynthRAD2023 Challenge Report. arXiv preprint arXiv:2403.08447; https://doi.org/10.48550/arXiv.2403.08447

**1.2 2D Conditional GAN**

In this implementation, a 2D conditional GAN (Pix2Pix) network is employed as follows. Let G: $X \to Y$ be a generator that translates images from domain $X$ to domain $Y$, and D be a discriminator network trained to distinguish between true and synthetized images in domain Y. The original Pix2Pix objective is:

$$\mathcal{L}(G, D) = \mathcal{L}_{GAN}(G, D) + \lambda \mathcal{L}_R(G) \qquad (1)$$

where $\mathcal{L}_{GAN}$ is the GAN loss and $\mathcal{L}_R$ is a regression loss measuring the difference between the output translation and the (aligned) ground truth. In the present implementation, three possible objective functions can be used for $\mathcal{L}_{GAN}$: classic (described, for example, in Goodfellow, I.: NIPS 2016 Tutorial: Generative Adversarial Networks, http://arxiv.org/abs/1701.00160, (2017) which is incorporated herein by reference); least squares (described, for example, in Mao, X., Li, Q., Xie, H., Lau, R.Y.K., Wang, Z., Smolley, S.P.: Least Squares Generative Adversarial Networks, http://arxiv.org/abs/1611.04076, (2017) which is incorporated herein by reference); and Hinge (described, for example, in Lim, J.H., Ye, J.C.: Geometric GAN, http://arxiv.org/abs/1705.02894, (2017) which is incorporated herein by reference). A L1 loss term is used for $\mathcal{L}_R$:

$$\mathcal{L}_R = E_{x,y} \|G(x) - y\|_1 \qquad (2)$$

The generator network is implemented with a ResUnet (described, for example, in Zhang, Z., Liu, Q., Wang, Y.: Road Extraction by Deep Residual U-Net. IEEE Geosci. Re-mote Sensing Lett. 15, 749-753 (2018) https://doi.org/10.1109/LGRS.2018.2802944, which is incorporated herein by reference). The number of layers and filters at each layer is fully parametrizable. The discriminator network is implemented similarly to the encoding part of the ResUnet. Both the generator and discriminator network can optionally use spectral normalization following each convolutional layer, and instance normalization is used in place of group normalization as shown in figure 4b.

Data augmentation can optionally be used, and may for example be randomly run on-the-fly during the training loop. Supported data augmentation includes: affine transformation, synthetic multiplicative bias field, blurring, sharpening, Gamma contrast change, linear intensity transform. Note that the affine transformation is applied to both MR and CT images. All other forms of data augmentation are applied to the MR images only.

The model is implemented in Python using the PyTorch library. Optimization is carried out using Adam with β1 = 0.5 and β2 = 0.999. A slow-moving exponential moving average (EMA) of the generator parameters was tracked during training (with α = 0.999, weights updated at every batch) and used as the final model for inference. A complete list of network hyperparameters is provided in Table 1 below.

## 1.3 Image Pre-Processing

The voxel resolution for model training may be chosen as 1x1x1 mm for brain model and 1x1x2.5 mm for the pelvis. If necessary, MR and CT images may be resampled to the model resolution and back to native resolution (during inference). MR and CT intensities are linearly rescaled to a range of [-1, +1], with a source range determined using percentiles for MR and a fixed source range of [-1000, +2200] HU or [-1000, +3000] HU to support metal artifacts. In the case study, models trained with the full range [-1000, +3000] were slightly less accurate because 1/4 of the intensity range [+2000, +3000] is reserved for unusual intensities. One strategy according to the present disclosure is to train one network with the full range and one with a narrower range, and use the content of the first network for intensities > 2200 (if any) and the content of the latter network otherwise.

During training, patches with a predefined sample size are randomly drawn from the training set images and randomly augmented. During inference, the test image is subdivided into overlapping patches and the model output combined via a weighted average - when combining the output of multiple overlapping patches, higher weight is given if a pixel is near the center of the patch and lower weight if the pixel is close to the edge of a patch.

**Table 1.** List of model hyperparameters

| Hyperparameter | Description | Recommended value |
|---|---|---|
| $\mathcal{L}_{GAN}$ | Type of adversarial loss function to use (either Classic, Least Squares or Hinge) | Least Squares |
| Learning rate | Learning rate for the generator (G) and discriminator (D). The learning rate will linearly decrease to zero starting when half the number of iterations has been completed. | G: 1e-4 D: 5e-5 |
| Num filters | Number of filters per layer in the generator (G) and discriminator (D) | G: 64,128,256,512 D: 64,128, 256,512,512 |
| Num disc updates | Number of discriminator updates per generator update | 2 |

(continued)

| Hyperparameter | Description | Recommended value |
|---|---|---|
| Spectral norm | Specify if spectral normalization layers are used in both the discriminator and generator True networks | True |
| L1 weight ($\lambda$) | Weight of L1 loss term | 50 |
| Voxel size | Voxel size in mm | Brain: 1x1x1 mm Pelvis: 1x1x2.5 mm |
| Sample size | The size of a sample (in voxels) used during training and inference. For a 2D network, the 3rd dimension is the channel dimension (c.f. Section 2.4) | 192x192x5 |
| Batch size | Number of samples per batch | 16 |
| Num batches per iteration | Number of batches per iteration | 300 |
| Num iterations | Total number of iterations | 2500 |
| Data augmentation | Option to turn on data augmentation | True |

### 1.4 Multi-Channel Implementation

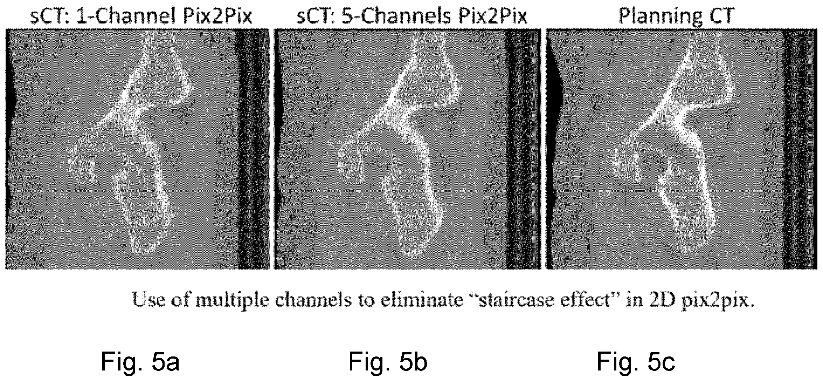

One problem with applying a 2D neural network independently on each axial slice of a 3D image is the so-called "staircase effect" that can appear in other orientations (sagittal and coronal). This is shown in figure 5a. One possible mitigation is to train a 3D network. However, this can lead to larger training and inference time, and higher GPU memory consumption in comparison to a 2D approach. Instead, a novel 2.5D approach is used, where five consecutive slices of data are supplied to the network, using the channel dimension. As illustrated in figure 5b, this approach significantly reduces the staircase effect without the drawbacks of a full 3D approach.

At training time, no change is required other than supplying randomly sampled groups of 5 consecutive slices of data. At inference time, the test image is subdivided into patches that overlap both in-plane and thru-plane. The model output is averaged as previously described in section 2.3.

### 2 Results

### 2.1 Strategy for hyperparameter tuning

During the training phase, the data was split for each anatomy (brain and pelvis) into a training set (75% of data) and a tuning set (25% of data). The training set is used to train the network and the tuning set is used for computing the image similarity metrics - mean absolute error (MAE), peak signal-to-noise (PSNR) and structure-similarity index (SSIM) - at the end of each training run. We started with an initial set of hyperparameters and then launched several trainings for the brain anatomy while varying one parameter at a time. Once a new set of best hyperparameters was found, this procedure was repeated a second time to obtain a final set of hyperparameters. Finally, a small hyperparameter search was performed on the pelvis anatomy, but no changes were found necessary other than the voxel size for keeping the training time reasonable.

### 2.2 Validation phase results

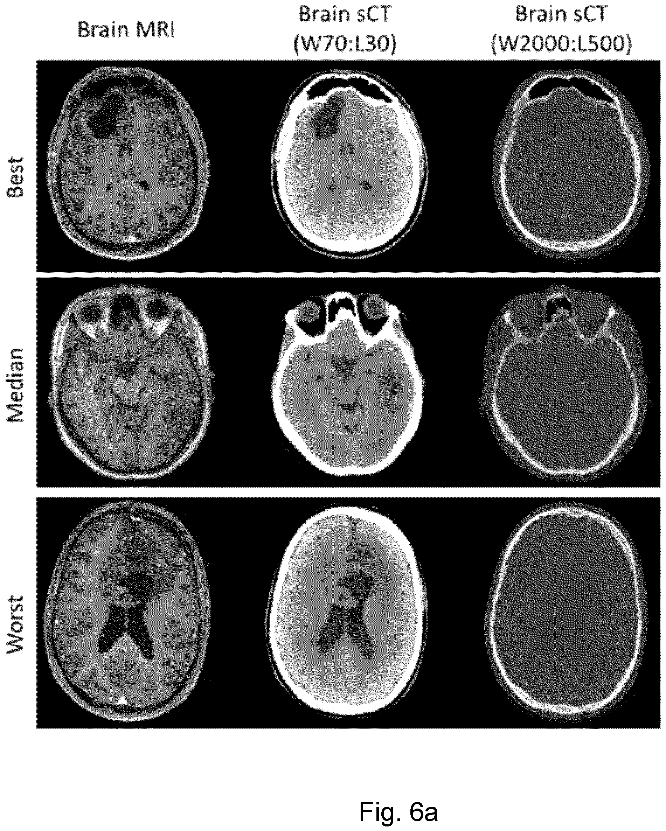

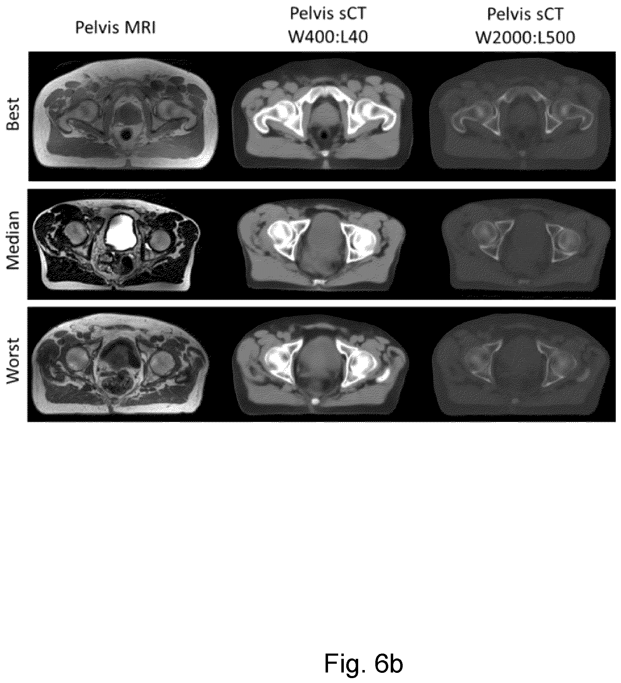

Once the validation phase started, brain and pelvis models were trained independently, using 100% of the training data and using best found hyperparameters. The tables of figures 6a and 6b show the results obtained during validation. The MAE is $64.27 \pm 14.15$ the PSNR is $28.64 \pm 1.77$ and the SSIM is $0.872 \pm 0.032$. Representative examples of brain and pelvis translations are shown in Fig. 6a. For each model, six networks were trained with the hyperparameters described in Table 1. The output of all six networks were combined with ensemble averaging. The average inference time for one network is approximately 20 seconds for brain images and 30 seconds for pelvis images on a workstation equipped with a NVIDIA V100 16GB GPU card. With ensemble averaging, the inference time scales linearly with the number of networks composing the model.

Figures 6a and 6b show representative examples of MR-to-sCT translation on the validation set. Images with best, median and worst MAE are shown. Synthetic CT images are displayed with different window/level settings to emphasis brain and/or soft tissue (middle image) and bone (right image).

**Table 2.** Final results of validation phase

| | MAE | PSNR | SSIM |
|---|---|---|---|
| **Mean** | 64.27 $\pm$14.15 | 28.64 $\pm$ 1.77 | 0.872 $\pm$ 0.032 |
| **Min** | 32.75 | 24.60 | 0.789 |
| **25pc** | 55.73 | 27.56 | 0.855 |
| **50pc** | 62.28 | 28.66 | 0.873 |
| **75pc** | 72.58 | 29.38 | 0.890 |
| **Max** | 109.88 | 34.58 | 0.969 |

### 2.3 Discussion

The results presented in section 3 indicate that a model based on the pix2pix architecture is suitable for MR-to-sCT image translation. Given that this is a paired method, accurate image registration is necessary between each MR/CT pairs. Rigid data alignment was used.

Different pulse sequences were used for the pelvis MRI (2/3 of the training data was T1-weighted, 1/3 was T2-weighted). Additional accuracy could be obtained by training different models for T1-weighted and T2-weighted MRI.

The maximum inference time was set to 15 minutes per image, which is acceptable for offline use, but not suitable for online adaptive workflows. In this work, ensemble averaging leads to small improvements on the overall image similarity metrics. However, the computational cost increases linearly with the number of networks in the model, with possibly very limited impact on the dosimetry.

For the pelvis tumor site, gas pockets in the rectum may not translate well into the sCT. This is because transient gas pocket information is not paired in the training data (i.e. MR and CT scans were not taken simultaneously). Moreover, they are difficult to visualize in MRI. Consequently, gas pockets may be hallucinated or missed out in the sCT. One mitigation is to mask all gas pockets in the training CTs with an average HU value. This may prevent the model from hallucinating air pockets during inference and, to some extent, would be consistent with some clinical workflows (large gas pockets are sometimes manually segmented and assigned an average HU/ED). However, during online imaging, gas pockets can provide useful information and radiation therapists may decide to wait for it to pass before starting the treatment.

The present application discloses the use of a 2.5D (multi-channel) pix2pix network for training MR-to-sCT models and discloses a case study which demonstrate results for both brain (T1-weighted) and pelvis (multi-contrast) anatomies. Separate models were provided in the case study for the different tumor sites as it led to superior image similarity metrics.

### *Generating sED images*

According to a second, related aspect of the present disclosure, a machine learning model can be trained to generate a synthetic image which contains the electron density information needed for radiotherapy treatment plan optimisation.

CT images contain and depict radiodensity information. Radiodensity and electron density (ED) are different concepts. Radiodensity refers to the degree to which tissue can prevent X-rays from passing through it. It is a measure of how much the tissue attenuates X-ray radiation. In contrast, electron density refers to the number of electrons per unit volume in a material. It is a measure of how electrons are distributed within a substance. The values are related but distinct from one another, and it is the electron density information for the relevant region of a patient's anatomy which is needed to plan radiotherapy treatment.

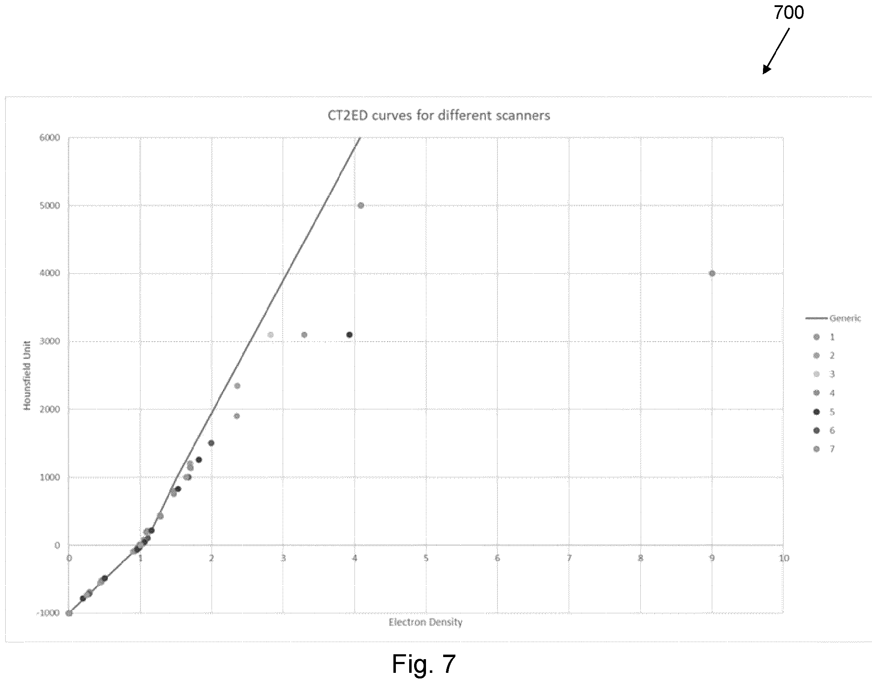

Figure 7 is a graph depicting "CT2ED" (or CT-ED) calibration data for different imaging scanners. To obtain data for the CT-ED calibration curve for a particular scanner, a tissue characterisation phantom or other object containing one or more materials with a known electron density is scanned by the CT scanner. In an example, the phantom may be sized and shaped like a human pelvis, but contains interchangeable rods made of various tissue equivalent materials. The phantom is placed in the imaging volume of a scanner, and CT images are taken. CT values in Hounsfield Units can be extracted from the resulting CT image or CT data. The graph 700 depicts data points for seven different scanners. As can be appreciated, the calibration data is distinct to each scanner. This is because many factors affect the CT values in CT data / images, for example the type of scanner, tube voltage, field of view, the reconstruction algorithm and software used, including artifact reduction and processing filters, etc..

Also shown in figure 7 is a "generic" CT-ED curve which shows a simple line which may be used when converting CT values in Hounsfield Units to electron density information in units of mass per unit volume, or to relative electron density. Here, the unit is e-/$a_0{}^3$ (electrons per cubic angstrom). As can be appreciated from the figure, it is possible to use the "generic" CT-ED curve when converting CT values to ED values, for example when converting a CT image to an ED image. While this gives a reasonably accurate ED value regardless of the type of scanner used, this approach will still introduce inaccuracies into the resulting ED data. The dosimetric impact of different CT number - ED calibration curves is discussed in: Das IJ, Cheng CW, Cao M, Johnstone PA. Computed tomography imaging parameters for inhomogeneity correction in radiation treatment planning. J Med Phys. 2016 Jan-Mar;41(1):3-11. doi: 10.4103/0971-6203.177277. PMID: 27051164; PMCID: PMC4795414.

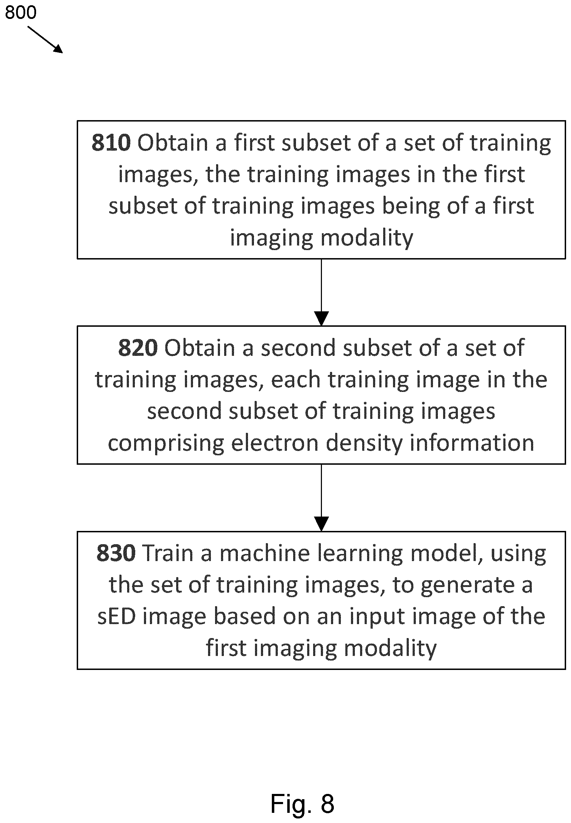

Figure 8 depicts a method 800 of training a machine learning model according to the present disclosure. Method 800 shares similarities with method 300 described above with respect to figure 3, and the skilled person will appreciate that aspects of each method can be used together, particularly in a specific implementation in which the model trained in method 800 is a generative model trained in a GAN.

At block 810, a first subset of a set of training images is obtained. These training images are of a first imaging modality. In the context of current radiotherapy workflows the most useful imaging modality for the first training data subset is MR images, however in principle the first imaging modality can be any imaging modality, for example ultrasound, Positron Emission Tomography (PET), Single Photon Emission Computed Tomography (SPECT), Dual-Energy X-ray Absorptiometry (DEXA), and the like.

Each image depicts an anatomical region of a patient. The images in the first subset may all depict the same patient, however in a more likely implementation the images in the first subset depict a variety of different patients. It is beneficial, but not essential, for the final trained model to be 'specialised' to a particular area of anatomy. Therefore, the image in the first subset may all depict a particular area of human anatomy, for example all the images in the first subset may depict a pelvic region, or the images may all depict the brain, etc.

Obtaining the first subset of images may comprise accessing the images from computer memory e.g. from a database of training data, for example, and/or obtaining the images may comprise acquiring the images using one or more imaging scanners.

Additional steps may be taken to ensure the images in the first subset are clean, standardized, and suitable to be input into the machine learning model. For example, the images may be 'cleaned' to remove duplicates and ensure they each show the relevant human anatomy, pixel values may be scaled and normalised, and the images may be re-sized to a standard size. These steps are known in the art and will not be discussed in further detail herein.

At block 820, a second subset of training images is obtained. Each training image in the second subset of training images comprises ED information. For example, each of the images in the second subset of training images may comprise a plurality of pixels or voxels each having a value representative of electron density. These training images are similar in form to a typical CT image, but rather than CT values in HU, these images have ED values in units of mass or electrons per unit volume. Alternatively, these images may consist of 2 channels: the first channel being the CT images in Hounsfield units and the second channel being the additional ED information , e.g. images for which each pixel or voxel is not only associated with a HU value but also an ED value. The term "synthetic electron density image" may be used to refer to an image that is computationally generated, in particular where the values are generated via an ML/AI model, and contains pixel intensity values directly related to electron density. To provide even more clarity: synthetic ED images, as used herein, are images generated via an AI/ML model. This differs from traditional ED images, which have values calculated from a CT by applying a calibration curve.

Obtaining the second subset of images may comprise generating the electron density information for each training image in the second subset of training images. This may be achieved using CT images and at least one CT-ED mapping (or 'calibration curve') of the type described above with respect to figure 7.

In an example, a generic CT-ED mapping is used to convert the HU units in a plurality of CT images to ED values, resulting in a plurality of ED images. The images in the resulting second subset of training images each thereby contain ED information. This approach works well, but the ED information can be made even more accurate if an ED-CT mapping is used which is associated with, i.e. specific to, the scanner which was used to generate the CT image.

Therefore, obtaining the second subset of training images at block 820 may comprise converting each of a plurality of CT images to training images comprising electron density information by using a plurality of CT-ED mappings, where, for each conversion, the mapping used is specific to the scanner used to generate the CT image. To aid understanding, it is helpful to consider two example images. In this implementation, a first and a second CT image is obtained. These images may be retrieved from memory, for example. The first CT image was generated using a first CT scanner, and the second CT image was generated using a second CT scanner. In this implementation, the method further comprises retrieving a first CT-ED calibration curve associated with the first CT scanner, and also retrieving a second (different) CT-ED calibration curve for the second scanner. First ED information is generated based on the first CT image by using a first CT-ED mapping, and second ED information is generated for the second CT image using the second CT-ED mapping. For example, the HU values in the first CT image are converted to ED values, and the HU values in the second image may be converted to ED values. The result is two training mages which comprise ED information, where the ED information has been generated using different CT-ED mappings. These training images therefore contain ED information which takes into account the features and parameters of the scanners used to acquire the original CT images, and which is therefore more accurate and more representative of the actual ED values in the anatomical structures and tissues depicted in the original CT images.

At block 830, a machine learning model is trained, using the set of training images (i.e. using both the first and the second subset of training images), to generate a sED image based on an input image of the first imaging modality. The sED image comprises electron density information in the same manner described above in relation to the second subset of images, but the data has been artificially generated. The sED may comprise, for example, a plurality of pixels or voxels each having a value representative of electron density.

The model can be trained in any of a number of ways known to the skilled person, including supervised translation using a neural network model such as a convolutional neural network model, supervised translation as part of a GAN network, and unsupervised translation e.g. as part of a CycleGAN network.

**Supervised translation**

In a first example, each image in the first subset may be paired with a corresponding image in the second subset. For example, each image of the first imaging modality may be paired with a corresponding image comprising ED data. The paired images may depict the same anatomical region, optionally may depict the same patient's anatomy, and may be registered to one another. A machine learning model such as a neural network model, for example a convolutional neural network model, may be trained in a known way using the paired images in the training set. The training process uses a supervised learning algorithm. The images in the first subset obtained at block 810 are the 'source' images and the training images in the second subset of are the 'target' images. During the training stage, the model weights are first initialised, and the model generates synthetic images using the source images as an input. The synthetic image is compared to the corresponding paired 'target' image. A suitable loss function may be a pixel-wise function, e.g. L1 or L2 pixel-level loss, for example. The network parameters such as the model weights are updated based on the results of the loss function in a known way.

In this way, a supervised learning algorithm can be used to train a machine learning model such as a neural network model to translate images of the first modality to images which comprise ED information.

**Supervised translation (GAN)**

While the above approach works well and can produce accurate results, using pixel-level loss alone can lead to blurry result. Accordingly, in an alternative implementation, the model may be trained in a supervised manner using a conditional GAN. The training method may be similar to that depicted and described above in relation to figure 3, and the GAN model and generator architecture may be similar to that depicted and described above in relation to figures 4a and 4b. An example of a suitable method is a pix2pix method and model, described elsewhere herein. The use of a GAN model is beneficial as the resulting synthetic images can be less blurry and more realistic.

The GAN is used to train a generator, using paired training data and in a known way that is described at a high level above with respect to figure 3, to output an sED image that resembles the images in the second subset based on an input image of the first modality. In more detail, in this implementation the GAN comprises a first generator model and a first discriminator model. The first generator model is trained to generate synthesised imaging data that resembles the training images in the second subset of training images based on input training images of the first subset of training images, and the first discriminator model is trained to discriminate between the synthesized imaging data generated by the first generative model and the training images in the second subset of training images.

**Unsupervised translation (CycleGAN)**

Alternatively, a CycleGAN model may be used. This is particularly beneficial when image pairs are not available, as it allows for unsupervised image translation. The cycleGAN is an extension of the GAN architecture. Two generator models and two discriminator models are trained simultaneously. This allows going from a first domain (e.g. images such as those in the first subset obtained at block 810) to a second domain (e.g. images such as those in the second subset obtained at block 820) and from the second domain back to the first domain while encouraging cycle consistency.

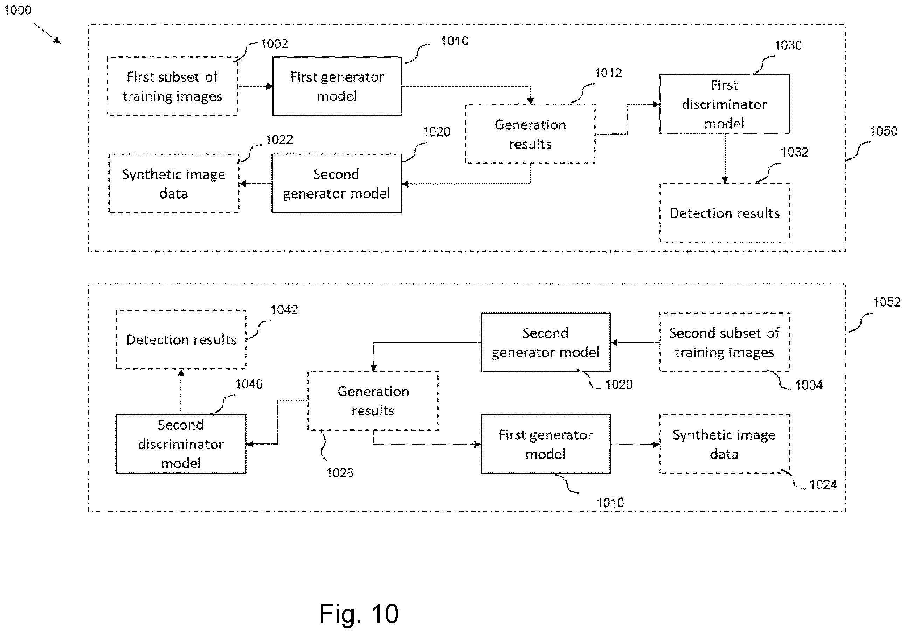

A CycleGAN model is described below in greater detail with respect to figure 10 but, in brief, in addition to a first generator and discriminator, the CycleGAN also comprises a second generative model trained to generate synthesised imaging data that resembles the training images in the first subset of training images based on input training images of the second subset of training images, and the second discriminator model is trained to discriminate between the synthesized imaging data generated by the second generative model and the training images in the first subset of training images.

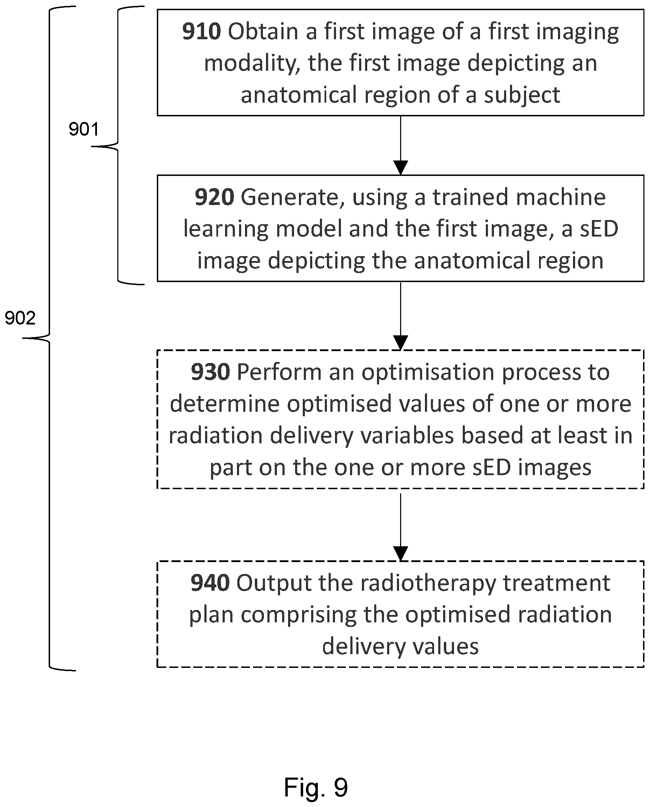

Once the model has been trained using an appropriate training method, it can be used to translate images of the first imaging modality, e.g. new MRI images not found in the training data, to images which correspond with the input image but which contain ED information, e.g. in line with the method 900 depicted in figure 9.

Figure 9 depicts a method 901 of generating one or more sED images according to the present disclosure, and a method 902 of optimising a radiotherapy treatment plan. Method 902 may be described as a method which enables adaptive radiotherapy (ART). Method 900 is similar to method 200 described above with respect to figure 2, and may be described as an implementation of method 200.

This application is primarily concerned with radiotherapy workflows. That is because a significant benefit of being able to generate an accurate synthetic ED image of a patient enables a radiotherapy treatment plan to be generated, or (re-)optimised, without the need to acquire a CT image of the patient. As a result, the plan can be re-optimised using the necessary ED information without the need to give the patient a dose of imaging radiation. However, it should be understood that ED images including synthetic ED images can be used in various fields, for example structural biology, material science, and chemistry. In an example, sED information may be used to calculate proton stopping power information for use in proton radiotherapy planning.

According to method 900, at block 910 a first image of the first imaging modality is obtained. The first image depicts an anatomical region of a subject and may for example be an MRI image. The MRI image may be a "daily MRI", e.g. an MR image taken on the day of treatment before a fraction of the patient's radiotherapy treatment is to be delivered according to an existing treatment plan. The image may be 2D or 3D. The image depicts a region of the patient's anatomy that is to undergo treatment, and therefore the image may contain one or more target regions including tumours, and one or more regions of healthy tissue including organs at risk (OARs).

At block 920, a trained machine learning model and the first image is used to generate a sED image depicting the same anatomical region. The sED image takes the form as described above e.g. with respect to figure 8, and therefore may comprise a plurality of pixels or voxels each having a value representative of electron density. This concludes method 901.

According to method 902, the method continues at block 930, and an optimisation process is performed to determine optimised values of one or more radiation delivery variables based at least in part on the one or more sED images.

To enable plan (re-)optimisation, the sED image may require segmentation data. For example, the input MR image may be contoured, i.e. contain segmentation data. When the MR image is converted to an sED image, the result is a sED image for which there is associated segmentation data indicating the various tissues and structures in the image.

Block 930 may comprise "re-" optimising an existing treatment plan, for example to account for changes in the patient's anatomy between the time of the original reference image and the day of treatment, or to account for changes since the time the previous fraction of treatment was delivered. For example, an initial radiotherapy treatment plan may have been generated based on a prior medical image, e.g. the reference CT image, during an offline planning stage. This initial radiotherapy treatment plan comprises initial values for the one or more radiation delivery variables. The initial values may have been optimised based on the prior medical image.

Performing the optimisation process at block 810 may comprises re-optimising these initial values for the one or more radiation delivery variables to generate optimised values based on the sED image generated at block 920. wherein performing the optimisation process comprises determining the optimised values of one or more radiation delivery variables based at least in part on one or more prescribed doses of radiation to regions of the patient's anatomy.

The radiation delivery variables, in part, define the treatment plan and may include, without limitation: the characteristics of the beam arrangement (e.g. the orientations of the beams and the number of beams directed toward the subject in each orientation); the positions of MLC leaves for each beam; the orientation of the MLC for each beam; and the weight of each beam.

The optimisation process may use any of a number of known optimisation algorithms. Performing the optimisation process at block 930 may comprise determining the optimised values of the radiation delivery variables based on planning or system constraints, for example at least in part based on one or more prescribed doses of radiation to regions of the patient's anatomy. Treatment planning constraints may include one or more of a prescribed dose for the target region, dose limits for healthy tissue (e.g., organs at risk). Other limitations may relate to system limitations e.g., fluence capabilities, MLC parameters, possible gantry speeds, table limits of movement, and the like.

At block 940, a radiotherapy treatment plan is outputted which comprises the optimised radiation delivery values. while not depicted in figure 9, the method may additional comprise delivering radiation to the subject according to the outputted radiotherapy treatment plan.

At a high-level, the "re-optimisation" process described at block 930 is a known process. Current online ART processes make use of daily MR images to re-optimise plans, for example using the "adapt-to-shape" workflow in Elekta's ™ MR-Linac, in which fluence or segment re-optimisation may be carried out. However, the current re-optimisation workflow relies on ED images acquired in a completely different way. In known techniques, the pre-treatment contours from the reference image are propagated by deformable registration onto the online 'daily' planning MR image. These propagated contours may then be edited by a radiation oncologist. Electron densities are assigned per structure based on the average ED value of the corresponding contour on the pre-treatment CT to enable the plan to be re-optimised on the online planning MRI and adjusted contours. The ED values are generated using the calibration curve for the specific CT scanner which took the reference CT. This is completely different to the presently disclosed methodologies, which comprise generating a synthetic ED image directly from an MR image.

The methods and systems described herein are advantageous in several ways. Generating a sED image using a trained machine learning model provides the information needed for radiotherapy plan (re-)optimisation in a manner which is extremely efficient. The process makes better use of clinician's time and enables treatment plans to be optimised more quickly. In addition, models trained according to the present methodology can generate sED images with ED information which is more accurate, i.e. more representative of the real electron density of the tissues and structures depicted in the anatomical region. In turn, this makes the treatment re-optimisation more accurate, resulting in more accurate and effective treatment.

By using different CT-ED mappings during the training data preparation phase, and in particular by using CT-ED curves which are specific to the scanner used to acquire the various images, the ED data in each ED image in the training data is made more accurate. The improved accuracy in the training data results in improved accuracy in the resulting model, and improved accuracy in the resulting the sED images.

In some implementations, both sCT and sED contrast may be needed. Both sCT and sED images could be produced using two separate models according to the presently disclosed method(s). In some implementations, however, a single model may be used with a 2-channel output layer: one for HU values and one for ED values. In this way, a model can be trained to take a single input image such as an MR image and generate a multi-layer output image with both a HU and electron density value for each voxel. In other words, the resulting model can generate both an sCT and an sED image via the same model.

## Implementation using a CycleGAN

The CycleGAN is a useful extension of a GAN, and is described below in connection with figure 10. Figure 10 illustrates training and use of CycleGAN 1000 for generating a sED image from a received first modality image according to some examples of the disclosure.

CycleGAN 1000 includes first generator model 1010, second generator model 1020, first discriminator model 1030 and second discriminator model 1040. CycleGAN 1000 may be divided into two portions, first portion 1050 and second portion 1052. In an example, in first portion 1050, first generator model 1010 may be trained to receive training image(s) 1002 of the first imaging modality, from the first subset of training images, and generate an image comprising synthetic electron density information as generation results 1012. Generation results 1012 may be provided to first discriminator model 1030. First discriminator model 1030 may classify the sED image as a real ED training image or a simulated ED training image and provide the classification as detection results 1032. The generation results 1012 and detection results 1032 may be fed back to first generator model 1010 and first discriminator model 1030 to adjust weights implemented by first generator model 1010 and first discriminator model 1030. For example, generation result 1012 (e.g., an sED image generated by first generator model 1010) may be used to calculate adversarial losses. Generation results 1012 (e.g., sED image) may also be concurrently provided to second generator model 1020. Second generator model 1020 may receive generation results 1012 and generate a simulated image of the first imaging modality as output. The simulated image may be referred to as cycle first imaging modality image 1022 or a cycle-MRI image in a specific example, and may be used to compute cycle losses to adjust weights of first/second generator model 1010/1020.

In an example, in second portion 1052, second generator model 1020 may be trained to receive a real image 1004 from the second subset of training images, i.e. a real ED image, and generate a synthetic image of the first imaging modality as generation results 1026. Generation results 1026 may be provided to second discriminator model 1040. Second discriminator model 1040 may classify the synthetic image as a real training image from the first subset of training mages, or a synthetic image of the first imaging modality, and provide the classification as detection results 1042. The generation results 1026 and detection results 1042 may be fed back to second generator model 1020 and second discriminator model 1040 to adjust weights implemented by second generator model 1020 and second discriminator model 1040. For example, generation result 1026 (e.g., a synthetic image of the first imaging modality generated by second generator model 1020) may be used to calculate adversarial losses. Generation results 1026 may also be concurrently provided to first generator model 1010. First generator model 1010 may receive generation results 1026 and generate an sED image as output. The sED image may be referred to as cycle ED image 1024 and may be used to compute cycle losses to adjust weights of first/second generator model 1010/1020.

In some examples, "adversarial losses" may account for the classification losses for the first and second discriminators 1030 and 1040. First and second discriminators 1030 and 1040 may classify whether the synthetic images have similar distributions as true images or not. For cycle-consistency losses, the losses are calculated between each pair of true and cycle images of the first imaging modality, and each pair of true and cycle ED images, respectively. For example, a first loss may be calculated between MR training image 1002 and cycle-MR image 1022 and a second loss may be calculated between real training ED image 1004 and cycle ED image 1024. The Cycle-MR image 1022 and Cycle-ED image 1024 may both be obtained by doing forward and backward cycles.

The CycleGAN 1000 may accordingly rely on a whole pool (or a plurality) of true or real first modality images 1002 and a whole pool (or a plurality) of true ED images 1004 to produce synthetic ED images (sED images) 1012 and synthetic MR images (sMR images) 1022. Based on "adversarial losses" and "cycle-consistency losses," CycleGAN 1000 may produce sharp synthetic ED images, which have similar image resolution as real ED images obtained via conversion from CT using a CT_ED mapping in the known manner.

In some examples, a processor (e.g., of system 1100) may apply image registration to register real ED training images to training MR images. This may create a one-to-one corresponding relationship between MR and ED images in the training data. This relationship may be referred to as paired or a pair of MR and ED images. In one example, CycleGAN 1000 may produce one or more sED images that preserve the exact same anatomy structure or substantially the same structure as in the corresponding MR images and that also have similar high image-quality as real MR images, including the pixel value accuracy. In some cases, these anatomy structures may be determined from mappings of anatomical areas which provide the metric representing similarity between two images. In an example, to preserve the pixel value accuracy of the sED image, corresponding to the MR image, additional constraints may be added to CycleGAN 1000. These constraints may include adding pixel value losses that represent pixel value loss between sED images and true ED images and between sMR images and true MR images.

**Second Case Study: Image translation using a CycleGAN**

**1 CycleGAN Overview**

Let $G : X \to Y$ and $F : Y \to X$ be two generators for the source-to-target and target-to-source directions, respectively. Let $D_X$ and $D_Y$ be two discriminators trained to distinguish between true and synthesized images in the two domains X and Y. The original CycleGAN objective is:

$$\mathcal{L}(G, F, D_X, D_Y) = \mathcal{L}_{GAN}(G, D_Y) + \mathcal{L}_{GAN}(F, D_X) + \lambda \mathcal{L}_{cycle}(G, F) \qquad (1)$$

where $\lambda$ is a constant. The goal of the $\mathcal{L}_{cycle}$ term is to constrain the generators to be cycle consistent: for image $x \sim X$, the image translation cycle should be able to recover the input, i.e. $x \to G(x) \to F(G(x)) \approx x$, and likewise for image $y \sim Y$, $y \to F(y) \to G(F(y)) \approx y$. The optimization problem is as follows:

$$G^*, F^*, D^*_X, D^*_Y = \underset{G,F}{\operatorname{argmin}} \underset{D_X D_Y}{\operatorname{argmax}} \mathcal{L}(G, F, D_X, D_Y) \qquad (2)$$

In practice, optimization consists of alternating updates to (2), in which the generator parameters are updated to minimize the loss function, and the discriminator parameters are updated to maximize the loss function. This case study focuses on a more general objective:

$$\mathcal{L}(G, F, D_X, D_Y) = \mathcal{L}_{GAN}(G, D_Y, X, Y) + L_{GAN}(F, D_X, Y, X) + \lambda_1 \mathcal{L}_{cycle}(G, F) + \lambda_2 \mathcal{L}_{struct}(G, F) \qquad (3)$$

where $\mathcal{L}_{struct}$ is an additional structure constraining loss function which penalizes differences between some aspect(s) of the input images and their immediate translations.

**1.1 L1 cycle-consistency**

The standard L1 cycle-consistency loss used in CycleGAN is given as follows:

$$L_{cycle}^{L1} = E_{x \sim X} \left[ \| \hat{x} - x \|_1 \right] + E_{y \sim Y} \left[ \| \hat{y} - y \|_1 \right] \qquad (4)$$

where $\hat{x} = F(G(x))$ is the reconstructed sample in domain X and $\hat{u} = G(F(x))$ is the reconstructed sample in domain Y.

**1.2 Perceptual cycle-consistency**

A perceptual loss can be defined by summing an L1 term over intermediate feature maps extracted by a feature extractor network. In practice, we use the discriminator networks for feature extraction, although pre-trained networks could also be used.

$$\mathcal{L}_{\text{cycle}}^{pL1} = \mathbb{E}_{x \sim X}\left[\Sigma_l \ \|(D_X(\hat{x}))_l - (D_X(x))_l\|_1\right] + \mathbb{E}_{y \sim Y}\left[\Sigma_l \ \|(D_Y(\hat{y}))_l - (D_Y(y))_l\|_1\right] \qquad (5)$$

where $(DX(x))l$ refers to the lth layer feature map extracted from input x by discriminator DX.

### 1.3 Structure-preserving contrastive loss

The noisy contrastive estimation (NCE) framework seeks to associate two signals: the 'query' and 'positive' samples, in *contrast* to the 'negative' samples. Herein we denote query, positive and N negative samples mapped to K-dimensional vectors as v, $v^+ \in R^K$ and $v^- \in R^{N \times K}$. Given these samples, the contrastive loss can be expressed as:

$$l(v, v^+, v^-) = -\log\left(\frac{\exp(\phi(v,v^+)/\tau)}{\exp\left(\phi(v,v^+)/\tau\right)+\sum_n \exp\left(\phi(v,v_n^-)/\tau\right)}\right) \qquad (6)$$

where $\phi(u, v)$ is the cosine similarity between u and v and $\tau$ is a temperature parameter to scale the distances between the query and other samples. Minimizing the contrastive loss is equivalent to finding a representation space (here $\mathbb{R}^k$ ) in which the query and positive samples have maximum mutual information.

Following CUT, we use the contrastive loss to encourage maximum local mutual information between the original and translated image. This requires a careful selection of the samples *v* to use in the contrastive loss.

For an input x ~ X, given a feature extractor network $D_X$, intermediate features from L layers are selected and sent through a small MLP network $H_X$ producing a stack of feature maps $\{z_l\}L = \{H_X(D_X(x))_l)\}_L$. Each spatial element s of a feature map represents local content of the image x. Given a query sample $z_l^s$ , we select N other randomly selected samples of the same image $z_l^{s/s}$ as negative samples. To obtain positive samples, we similarly encode the translated image G(x) into $\{\hat{z}_l\}L = \{H_Y(D_Y(G_x)_l)_l\}_L$

For the mapping in the X → Y direction we have:

$$\mathcal{L}_{\text{struct}}^{X \to Y} = \mathbb{E}_{x \sim X} \Sigma_l \ \Sigma_s \ l\left(\hat{z}_l^s, z_l^s, z_l^{S\backslash s}\right) \qquad (7)$$

This loss is minimized when there is maximal mutual information between pairs of positive samples $\{z_l^s, \hat{z}_l^s\}$ . For the mapping in the *Y → X* direction we similarly have:

$$\mathcal{L}_{\text{struct}}^{Y \to X} = \mathbb{E}_{y \sim Y} \Sigma_l \ \Sigma_s \ l\left(\hat{z}_l^s, z_l^s, z_l^{S\backslash s}\right) \qquad (8)$$

wher $\{z_l\}_L = \{H_Y(D_Y(y)_l)\}_L$ and $\{\hat{z}l\}L = \{H_X(D_X(F(y))_l)\}_L$. The total structure-preserving loss is given by:

$$\mathcal{L}_{\text{struct}} = \mathcal{L}_{\text{struct}}^{X \to Y} + \mathcal{L}_{\text{struct}}^{Y \to X} . \qquad (9)$$

### 1.4 Self-attention layers

We experiment with adding scaled dot-product attention layers in the generator network. Such layers are integral parts of Transformer models. Given an input x, it is reshaped into a vector and projected three times using learned linear mappings into the queries $Q \in \mathbb{R}^{N \times d_k}$ , the keys $K \in \mathbb{R}^{N \times d_k}$ and the values $V \in \mathbb{R}^{N \times d_v}$ . The attended output A is computed as:

$$A(Q, K, V) = \text{softmax}\left(\frac{QK^T}{\sqrt{d_k}}\right)V \qquad (10)$$

where A can be projected to the original dimensionality if $d_v$ is not the same as the input channel dimensionality. Finally, a residual connection is added, i.e. x ← x + *A(x)*.

**1.5 Discriminator network**

The classical CycleGAN method uses a discriminator network with an encoder structure. The network consists of repeated applications of standard convolutional layers with stride 2, each reducing the size of the input by a factor of 2 along each spatial dimension. In the present case study, for consistency with the ResUNet generator, swap the standard convolutional layers of the discriminator are swapped for residual blocks. It is also contemplated to reduce the encoder discriminator with a full ResUNet model.

**2 Pix2Pix Overview**

Pix2Pix is a supervised image translation method which requires well-aligned input/output pairs. Let $G : X \rightarrow Y$ be a generator that translates images from domain $X$ to domain $Y$, and $D$ be a discriminator network trained to distinguish between true and synthesized images in the domain $Y$. The original Pix2Pix objective is:

$$\mathcal{L}(G, D) = \mathcal{L}_{GAN}(G, D) + \lambda \mathcal{L}_R(G) \qquad (11)$$

where $\mathcal{L}_R$ is a regression loss measuring the difference between the output translation and the (aligned) ground truth:

$$\mathcal{L}_R = \mathbb{E}_{x,y} \| G(x) - y \|_1. \qquad (12)$$

As in the case of CycleGAN, both the generator and discriminator networks are optimized alternatively. In the experiments below, we consider several of the same modifications applied to the CycleGAN model (e.g. using a perceptual loss instead of the *L1* term in equation 12, using self-attention layers, and using a ResUNet discriminator).

It is possible to use a shared feature extractor for encoding images x ~ X and y ~ Y. Herein, instead better translation results are obtained by leveraging domain-specific encoders (here, domain-specific discriminators).

**3 Experiments and Results**

**3.1 Data and initial setup**

47 head and neck patients were considered, with both a 6-min T2w MRI and a CT scan. For each patient, the MR and CT images were rigidly aligned. The rigidly aligned datasets are directly used as training data for the CycleGAN method. For the Pix2Pix method, the training data were additionally deformably aligned. For both methods, test data (corresponding pairs of MR/CT images) are deformably aligned. Following alignment, the registered pairs are both resampled to an isotropic voxel size of 1 mm$^3$. All experiments below report experiments using a 3-fold cross-validation approach.

**3.2 Model configuration**

Both training and inference are done on images resampled to an isotropic voxel size of 1.5 mm$^3$. After inference, the model output is resampled to 1 mm$^3$. For all experiments, we consider a 2D approach and draw samples from the axial plane of size [192, 192] voxels.

For both CycleGAN and Pix2Pix methods, we use a ResUNet generator. All models (in both the generator and discriminator) use spectral normalization following each convolutional layer, and instance normalization is used in place of group normalization.

Optimization is carried out using Adam with $\beta1 = 0.5$ and $\beta2 = 0.999$. A slow-moving exponential moving average (EMA) of the generator parameters is tracked during training (with a = 0.999, weights updated at every batch) and used as the final model for inference.

Finally, for self-attention, the layers are attached to the intermediate outputs of the generator decoder only (e.g. in the two layers producing feature maps 64 and 128).

**3.3 CycleGAN Results and Key model configuration parameters**

A number of mechanisms to increase the performance of the CycleGAN baseline model were implemented: (1) addition of structure-preserving contrastive loss, (2) addition of self-attention layers in the generator decoder, (3) replacement of standard L1 cycle-consistency loss with perceptual cycle-consistency loss, and (4) replacement of standard encoder discriminator with a ResUNet discriminator. Note that the CycleGAN baseline uses an L1 cycle loss and an encoder discriminator (ED). Including the structure-preserving contrastive loss had a large positive impact on performance.

Table 1: Key model configuration parameters used in the second case study

|  | Value or type | Comments |
|---|---|---|
| $\mathcal{L}_{GAN}$ | Least squares | |
| Generator architecture | ResUNet | |
| Generator feature maps | [32, 64, 128, 256] | |
| Generator learning rate | *1e - 4* | |
| Discriminator architecture | Stack of ResBlocks | Encoder-only model |
| Discriminator feature maps | [32, 64, 128, 256] | |
| Discriminator learning rate | *1e - 4* | 5e-5 if using ResUNet discriminator |
| Cycle-consistency weight $\lambda_{cycle}$ | 10 | |
| Regression weight $\lambda_R$ | 50 | |
| Perceptual cycle consistency weight $\lambda_{cycle}$ | 10 | |
| Structure preserving weight $\lambda_{struct}$ | 1 | Contrastive loss |

Figure 11 illustrates a block diagram of one implementation of a system 1100, for example a radiotherapy system. The radiotherapy system 1100 comprises a computing system 1110 within which a set of instructions, for causing the computing system 1110 to perform any one or more of the methods discussed herein, may be executed.

The computing system 1110 shall be taken to include any number or collection of machines, e.g. computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 1110 includes controller circuitry 1111 and a memory 1113 (e.g., read-only memory (ROM), flash memory, dynamic random access memory (DRAM) such as synchronous DRAM (SDRAM) or Rambus DRAM (RDRAM), etc.). The memory 1113 may comprise a static memory (e.g., flash memory, static random access memory (SRAM), etc.), and/or a secondary memory (e.g., a data storage device), which communicate with each other via a bus (not shown).

Controller circuitry 1111 represents one or more general-purpose processors such as a microprocessor, central processing unit, accelerated processing units, or the like. More particularly, the controller circuitry 1111 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 1111 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 1111 is configured to execute the processing logic for performing the operations and steps discussed herein.

The computing system 1110 may further include a network interface circuitry 1118. The computing system 1110 may be communicatively coupled to an input device 1120 and/or an output device 1130, via input/output circuitry 1117. In some implementations, the input device 1120 and/or the output device 1130 may be elements of the computing system 1110. The input device 1120 may include an alphanumeric input device (e.g., a keyboard or touchscreen), a cursor control device (e.g., a mouse or touchscreen), an audio device such as a microphone, and/or a haptic input device. The output device 1130 may include an audio device such as a speaker, a video display unit (e.g., a liquid crystal display (LCD) or a cathode ray tube (CRT)), and/or a haptic output device. In some implementations, the input device 1120 and the output device 1130 may be provided as a single device, or as separate devices.

In some implementations, computing system 1110 includes training circuitry 1118. The training circuitry 1118 is configured to train a method of generating a synthetic image; for example using the method described above with respect to figure 3. The model may comprise a deep neural network (DNN), such as a convolutional neural network (CNN) and/or recurrent neural network (RNN). Training circuitry 1118 may be configured to access training data and/or testing data from memory 1113 or from a remote data source, for example via network interface circuitry 1115. In some examples, training data and/or testing data may be obtained from an external component, such as image acquisition device 1140 and/or treatment device 1150. In some implementations, training circuitry 1118 may be used to update, verify and/or maintain the model for generating a synthetic image.

In some implementations, the computing system 1110 may comprise image processing circuitry 1119. Image processing circuitry 1119 may be configured to process image data 1180 (e.g. images, or imaging data), such as medical images obtained from one or more imaging data sources, a treatment device 1150 and/or an image acquisition device 1140. Image processing circuitry 1119 may be configured to process, or pre-process, image data. For example, image processing circuitry 1119 may convert received image data into a particular format, size, resolution or the like. In some implementations, image processing circuitry 1119 may be combined with controller circuitry 1111.

In some implementations, the radiotherapy system 1100 may further comprise an image acquisition device 1140 and/or a treatment device 1150, such as those disclosed herein in the examples of Figure 1. The image acquisition device 1140 and the treatment device 1150 may be provided as a single device. In some implementations, treatment device 1150 is configured to perform imaging, for example in addition to providing treatment and/or during treatment.

Image acquisition device 1140 may be configured to perform positron emission tomography (PET), computed tomography (CT), but in a preferred implementation is configured to generate magnetic resonance (MR) images. Image acquisition device 1140 may be configured to output image data 1180, which may be accessed by computing system 1110. Treatment device 1150 may be configured to output treatment data 1160, which may be accessed by computing system 1110.

Computing system 1110 may be configured to access or obtain treatment data 1160, planning data 1170 and/or image data 1180. Treatment data 1160 may be obtained from an internal data source (e.g. from memory 1113) or from an external data source, such as treatment device 1150 or an external database. Planning data 1170 may be obtained from memory 1113 and/or from an external source, such as a planning database. Planning data 1170 may comprise information obtained from one or more of the image acquisition device 1140 and the treatment device 1150.

The various methods described above may be implemented by a computer program. The computer program may include computer code (e.g. instructions) 1210 arranged to instruct a computer to perform the functions of one or more of the various methods described above. The steps of the methods described above may be performed in any suitable order. The computer program and/or the code 1210 for performing such methods may be provided to an apparatus, such as a computer, on one or more computer readable media or, more generally, a computer program product 1200)), depicted in Figure 8. The computer readable media may be transitory or non-transitory. The one or more computer readable media 1200 could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions 1210 may also reside, completely or at least partially, within the memory 1113 and/or within the controller circuitry 1111 during execution thereof by the computing system 1110, the memory 1113 and the controller circuitry 1111 also constituting computer-readable storage media.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as " receiving", "determining", "comparing ", "enabling", "maintaining," "identifying" or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the present description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

The following items are disclosed herein, which relate to the first aspect of the disclosure:

1. A computer-implemented method for generating a synthetic image based on a first image of a first imaging modality, the method comprising:

receiving the first image, the first image depicting an anatomical region of a subject; and
generating, using a generative model, a synthetic image corresponding to the first image,
wherein the synthetic image corresponds to a second imaging modality.

2. The method of item 1, wherein the first imaging modality is a magnetic resonance (MR) image.

3. The method of any preceding item, wherein the second imaging modality is any of computed tomography (CT) and cone-beam computed tomography (CBCT); and/or wherein the synthetic image is a synthetic CT (sCT) image.

4. The method of any preceding item, wherein the generative model has been trained via a generative adversarial network (GAN).

5. The method of item 4, wherein the GAN comprises a first generator convolutional network and a first discriminator convolutional network.

6. The method of item 5, wherein the first generator convolutional network is trained to generate synthetic images based on input training images of the first imaging modality, and the first discriminator network is configured to discriminate between synthesized imaging data corresponding to the second imaging modality and actual imaging data corresponding to the second imaging modality.

7. The method of any preceding item, wherein the first image is a three-dimensional (3D) image comprising a plurality of voxels; and wherein generating the synthetic image comprises providing, as an input to the generative model, an image sample comprising a plurality of consecutive 2D image patches; wherein the consecutive 2D image patches in the image sample are arranged perpendicular to a first image axis and each 2D image patch depicts a 2D region in the first image.

8. The method of item 7, the method further comprising: providing a first set of image samples as an input to the generative model to obtain a model output for each image sample, and aggregating the model outputs to generate the synthetic image.

9. The method of item 8, wherein the first set of image samples comprises a first image sample with a first plurality of consecutive 2D image patches and a second image sample with a second plurality of consecutive 2D image patches, wherein the first and second plurality of consecutive 2D image patches overlap with one another such that the voxels of a first subset of voxels are comprised within both the first and the second image sample.

10. The method of item 8 or item 9 wherein, in an optional embodiment, the method further comprising providing a second set of image samples to the generative model, wherein the image samples in the second set of image samples each comprise consecutive 2D image patches arranged perpendicular to a second image axis.

11. The method of item 10, wherein the second set of image samples comprises a third image sample comprising a third plurality of consecutive 2D image patches, wherein the third plurality of consecutive 2D image patches and the first plurality of consecutive 2D image patches overlap with one another such that the voxels of a second subset of voxels are comprised within both the first and the third image sample.

12. The method of any of items 8 to 11, wherein the method further comprises: subdividing the first image into the first and/or the second set of image samples.

13. The method of any of items 8 to 12, wherein the model outputs for each image sample comprise a synthetic CT value for each voxel of the image sample.

14. The method of item 13, wherein aggregating the model outputs comprises, for each voxel of the first and/or the second image samples, calculating an average of the synthetic CT values available for that voxel.

15. The method of item 14, wherein the average is a weighted average, the weight of each synthetic CT value being based on a proximity from a centre of its image sample.

16. The method of any preceding item, wherein the generative model comprises a ResUnet generator.

17. A computer-implemented method for training a generative model to generate a synthetic image, the method comprising performing a process comprising:

receiving training data comprising images of a first imaging modality and images of a second imaging modality; and
providing, as an input to a generative model in a generative adversarial network (GAN), imaging data based on the images of the first imaging modality, and receiving estimated synthetic imaging data as an output from the generative model;
determine, using a discriminative model in the GAN, at least one difference between the estimated synthetic imaging data and real imaging data based on the images of the second imaging modality; and
update one or more parameters of the generative model based on the determined at least one difference;
the method further comprising iteratively performing the process until, when a stopping criterion is reached, the method comprises outputting the trained model;
wherein, after being trained, the generative model is configured to receive an image of the first modality as an input, and output a synthetic image corresponding to the second imaging modality.

18. A system for generating a synthetic image, the system comprising:

processing circuitry comprising at least one processor; and
a computer-readable medium comprising instructions, which when executed by the at least one processor, cause the processor to perform the method of any preceding item.

19. A computer-readable medium comprising computer-readable instructions which, when executed by at least one processor, cause the at least one processor to perform the method of any of items 1 to 17.

The following items are disclosed herein, which relate to the second aspect of the disclosure:

1. A computer-implemented method for generating one or more synthetic electron density, sED, images, the method comprising:

obtaining a first image of a first imaging modality, the first image depicting an anatomical region of a subject; and

generating, using a trained machine learning model and the first image, a sED image depicting the anatomical region;

wherein the trained machine learning model has been trained using a set of training images comprising a first subset of training images of the first imaging modality, and a second subset of training images in which each training image comprises electron density, ED, information.

2. The method of item 1, wherein the first imaging modality is magnetic resonance, MR, imaging.

3. The method of item 1 or item 2, wherein the sED image comprises a plurality of pixels or voxels each having a value representative of electron density.

4. The method of any preceding item, wherein each of the images in the second subset of training images comprises a plurality of pixels or voxels each having a value representative of electron density.

5. The method of any preceding item, wherein the electron density information for each image in the second subset of training images has been generated based on a respective computed tomography, CT, image using at least one CT-ED mapping.

6. The method of item 5, wherein the second subset of training images comprises a first training image and a second training image, wherein the first training image comprises first ED information generated from a first CT image using a first CT-ED mapping, and the second training image comprises second ED information generated from a second CT image obtained using a second CT imaging device using a second, different CT-ED mapping.

7. The method of item 6, wherein the first training image has been obtained using a first CT imaging device and the first CT-ED mapping is specific to the first CT imaging device, and the second training image has been obtained using a second CT imaging device and the second CT-ED mapping is specific to the second CT imaging device.

8. The method of any preceding item, wherein the trained machine learning model is a generative model, wherein the generative model has been trained via a generative adversarial network (GAN).

9. The method of item 8, wherein the generative model is a first generator model, and the GAN comprises the first generator model and a first discriminator model, the first generator model trained to generate synthesised imaging data that resembles the training images in the second subset of training images based on input training images of the first subset of training images, and the first discriminator model trained to discriminate between the synthesized imaging data generated by the first generative model and the training images in the second subset of training images.

10. The method of item 9, wherein the generative adversarial network is a cycle generative adversarial network, CycleGAN, and the CycleGAN further comprises:
a second generative model trained to generate synthesised imaging data that resembles the training images in the first subset of training images based on input training images of the second subset of training images, and a second discriminator model trained to discriminate between the synthesized imaging data generated by the second generative model and the training images in the first subset of training images.

11. A method for training a machine learning model to generate one or more synthetic electron density (sED) images using a set of training images, the set of training images comprising a first and a second subset of training images, each training image in the set of training images depicting an anatomical region of one or more patients, the method comprising:

obtaining the first subset of training images, the first subset of training images being of a first imaging modality;
obtaining the second subset of training images, each training image in the second subset of training images comprising electron density, ED, information; and
training the machine learning model, using the set of training images, to generate a sED image based on an input image of the first imaging modality.

12. The method of item 11, wherein the first imaging modality is magnetic resonance, MR, imaging.

13. The method of item 11 or item 12, wherein the sED comprises a plurality of pixels or voxels each having a value representative of electron density.

14. The method of any of item 11-13, wherein each of the images in the second subset of training images comprises a plurality of pixels or voxels each having a value representative of electron density.

15. The method of any of item 11-14, further comprising generating the electron density information for each training image in the second subset of training images based on a respective computed tomography, CT, image using at least one CT-ED mapping.

16. The method of item 15, wherein the second subset of training images comprises a first training image and a second training image, and the method further comprises:

generating first ED information for the first training image from a first CT image using a first CT-ED mapping; and generating second ED information for the second training image from a second CT image using a second, different CT-ED mapping.

17. The method of item 16, wherein the first CT image was obtained using a first CT imaging device and the first CT-ED mapping is specific to the first CT imaging device, and the second CT image was obtained using a second CT imaging device and the second CT-ED mapping is specific to the second CT imaging device.

18. The method of any of item 11 to 17, wherein the machine learning model is a generative model in a generative adversarial network (GAN).

19. The method of item 18, wherein the GAN comprises a first generator model and a first discriminator model, the first generator model trained to generate synthesised imaging data that resembles the training images in the second subset of training images based on input training images of the first subset of training images, and the first discriminator model trained to discriminate between the synthesized imaging data generated by the first generative model and the training images in the second subset of training images.

20. The method of item 19, wherein the generative adversarial network is a cycle generative adversarial network, CycleGAN, and the CycleGAN further comprises:
a second generative model trained to generate synthesised imaging data that resembles the training images in the first subset of training images based on input training images of the second subset of training images, and a second discriminator model trained to discriminate between the synthesized imaging data generated by the second generative model and the training images in the first subset of training images.

21. A trained machine learning model training according to the method of any of item 11 to 20.

22. A computed-implemented method for optimising a radiotherapy treatment plan, comprising:

obtaining a first image of a patient, the first image being of a first imaging modality;
generating one or more synthetic electron density (sED) images using a trained machine learning model and the first image according to the method of any preceding claim;
performing an optimisation process to determine optimised values of one or more radiation delivery variables based at least in part on the one or more sED images; and
outputting the radiotherapy treatment plan comprising the optimised radiation delivery values.

23. The method of item 22, further comprising receiving an initial radiotherapy treatment plan for a patient generated based on a prior medical image, the initial radiotherapy treatment plan comprising initial values for the one or more radiation delivery variables; and
wherein performing the optimisation process comprises re-optimising the initial values for the one or more radiation delivery variables to generate the optimised values of the one or more radiation delivery variables based on the one or more sED images.

24. A computer-readable medium comprising computer-executable instructions which, when executed by one or more processors, cause the one or more processors to perform the method of any of items 1 to 23.

25. A system comprising one or more processors and a computer-readable medium comprising computer-executable instructions which, when executed by the one or more processors, cause the one or more processors to perform the method of any of items 1 to 23.

**Claims**

1.  A computer-implemented method for generating one or more synthetic electron density, sED, images, the method comprising:

obtaining a first image of a first imaging modality, the first image depicting an anatomical region of a subject; and

generating, using a trained machine learning model and the first image, a sED image depicting the anatomical region;

wherein the trained machine learning model has been trained using a set of training images comprising a first subset of training images of the first imaging modality, and a second subset of training images in which each training image comprises electron density, ED, information.

2. The method of claim 1, wherein the first imaging modality is magnetic resonance, MR, imaging.

3. The method of claim 1 or claim 2, wherein the sED image comprises a plurality of pixels or voxels each having a value representative of electron density.

4. The method of ant preceding claim, wherein each of the images in the second subset of training images comprises a plurality of pixels or voxels each having a value representative of electron density.

5. The method of any preceding claim, wherein the electron density information for each image in the second subset of training images has been generated based on a respective computed tomography, CT, image using at least one CT-ED mapping.

6. The method of claim 5, wherein the second subset of training images comprises a first training image and a second training image, wherein the first training image comprises first ED information generated from a first CT image using a first CT-ED mapping, and the second training image comprises second ED information generated from a second CT image obtained using a second CT imaging device using a second, different CT-ED mapping.

7. The method of claim 6, wherein the first training image has been obtained using a first CT imaging device and the first CT-ED mapping is specific to the first CT imaging device, and the second training image has been obtained using a second CT imaging device and the second CT-ED mapping is specific to the second CT imaging device.

8. A method for training a machine learning model to generate one or more synthetic electron density (sED) images using a set of training images, the set of training images comprising a first and a second subset of training images, each training image in the set of training images depicting an anatomical region of one or more patients, the method comprising:

obtaining the first subset of training images, the first subset of training images being of a first imaging modality;
obtaining the second subset of training images, each training image in the second subset of training images comprising electron density, ED, information; and
training the machine learning model, using the set of training images, to generate a sED image based on an input image of the first imaging modality.

9. The method of any of claim 8, further comprising generating the electron density information for each training image in the second subset of training images based on a respective computed tomography, CT, image using at least one CT-ED mapping.

10. The method of claim 9, wherein the second subset of training images comprises a first training image and a second training image, and the method further comprises:

generating first ED information for the first training image from a first CT image using a first CT-ED mapping; and
generating second ED information for the second training image from a second CT image using a second, different CT-ED mapping.

11. The method of claim 10, wherein the first CT image was obtained using a first CT imaging device and the first CT-ED mapping is specific to the first CT imaging device, and the second CT image was obtained using a second CT imaging device and the second CT-ED mapping is specific to the second CT imaging device.

12. A trained machine learning model training according to the method of any of claims 8 to 11.

13. A computed-implemented method for optimising a radiotherapy treatment plan, comprising:

obtaining a first image of a patient, the first image being of a first imaging modality;

generating one or more synthetic electron density (sED) images using a trained machine learning model and the first image according to the method of any preceding claim;

performing an optimisation process to determine optimised values of one or more radiation delivery variables based at least in part on the one or more sED images; and

outputting the radiotherapy treatment plan comprising the optimised radiation delivery values.

14. A computer-readable medium comprising computer-executable instructions which, when executed by one or more processors, cause the one or more processors to perform the method of any of claims 1 to 13.

15. A system comprising one or more processors and a computer-readable medium comprising computer-executable instructions which, when executed by the one or more processors, cause the one or more processors to perform the method of any of claims 1 to 13.

Fig. 1

200

```
┌─────────────────────────────────────────────┐
│                    210                       │
│        Receive a first image depicting an    │
│          anatomical region of a subject      │
└─────────────────────────────────────────────┘
                        │
                        ▼
┌─────────────────────────────────────────────┐
│                    220                       │
│        Generate, using a generative model, a │
│      synthetic image corresponding to the first │
│                    image                     │
└─────────────────────────────────────────────┘
```

# Fig. 2

**300**

**310**
Receive training data comprising images of a first imaging modality and images of a second imaging modality

**320**
Train generative model to generate synthetic images

**330**
Train discriminative model to discriminate between real and synthetic images

**350**
Output trained generative model

**360**
Utilise trained generative model to generate a synthetic image according to flowchart of figure 2

# Fig. 3

Fig. 4a

EP 4 513 425 A1

Fig. 4b

sCT: 1-Channel Pix2Pix   sCT: 5-Channels Pix2Pix   Planning CT

Use of multiple channels to eliminate "staircase effect" in 2D pix2pix.

Fig. 5a                    Fig. 5b                    Fig. 5c

Fig. 6a

Fig. 6b

700

CT2ED curves for different scanners

Fig. 7

800

**810** Obtain a first subset of a set of training images, the training images in the first subset of training images being of a first imaging modality

**820** Obtain a second subset of a set of training images, each training image in the second subset of training images comprising electron density information

**830** Train a machine learning model, using the set of training images, to generate a sED image based on an input image of the first imaging modality

Fig. 8

**910** Obtain a first image of a first imaging modality, the first image depicting an anatomical region of a subject

**920** Generate, using a trained machine learning model and the first image, a sED image depicting the anatomical region

**930** Perform an optimisation process to determine optimised values of one or more radiation delivery variables based at least in part on the one or more sED images

**940** Output the radiotherapy treatment plan comprising the optimised radiation delivery values

901

902

Fig. 9

Fig. 10

Fig. 11

Fig. 12

| | Europäisches Patentamt European Patent Office Office européen des brevets | **EUROPEAN SEARCH REPORT** | **Application Number** EP 24 19 6019 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>A | US 2022/225955 A1 (SIVERSSON CARL [SE]) 21 July 2022 (2022-07-21) <br> * abstract * <br> * paragraphs [0001], [0002], [0009], [0029] - [0032], [0036], [0039], [0044] * <br> * claim 1 * | 1-5,8,9, 12-15 <br> 6,7,10, 11 | INV. G06T5/60 G06T7/00 |
| A | LI YAFEN ET AL: "CT synthesis from MRI images based on deep learning methods for MRI-only radiotherapy", 2019 INTERNATIONAL CONFERENCE ON MEDICAL IMAGING PHYSICS AND ENGINEERING (ICMIPE), IEEE, 22 November 2019 (2019-11-22), pages 1-6, XP033773922, DOI: 10.1109/ICMIPE47306.2019.9098190 [retrieved on 2020-05-21] * the whole document * | 1-15 | |
| A | JIE FU ET AL: "Male pelvic synthetic CT generation from T1-weighted MRI using 2D and 3D convolutional neural networks", ARXIV.ORG, CORNELL UNIVERSITY LIBRARY, 201 OLIN LIBRARY CORNELL UNIVERSITY ITHACA, NY 14853, 1 March 2018 (2018-03-01), XP081376934, DOI: 10.1002/MP.13672 * the whole document * | 1-15 | **TECHNICAL FIELDS SEARCHED (IPC)** <br><br> G06T |
| A | US 2020/034948 A1 (PARK CHUNJOO [US] ET AL) 30 January 2020 (2020-01-30) * the whole document * | 1-15 | |

The present search report has been drawn up for all claims

| Place of search | Date of completion of the search | Examiner |
|---|---|---|
| Munich | 8 January 2025 | Eveno, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 6019

This annex lists the patent family members relating to the patent documents cited in the above-mentioned European search report.
The members are as contained in the European Patent Office EDP file on
The European Patent Office is in no way liable for these particulars which are merely given for the purpose of information.

08-01-2025

| Patent document cited in search report | Publication date | Patent family member(s) | | Publication date |
|---|---|---|---|---|
| US 2022225955 A1 | 21-07-2022 | EP | 3958965 A1 | 02-03-2022 |
| | | JP | 7572733 B2 | 24-10-2024 |
| | | JP | 2022530108 A | 27-06-2022 |
| | | US | 2022225955 A1 | 21-07-2022 |
| | | US | 2024298990 A1 | 12-09-2024 |
| | | WO | 2020218967 A1 | 29-10-2020 |
| US 2020034948 A1 | 30-01-2020 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **THUMMERER A** ; **VAN DER BIJL E** ; **GALAPON JR A** ; **VERHOEFF JJ** ; **LANGENDIJK JA** ; **BOTH S** ; **VAN DEN BERG CAT** ; **MASPERO M.** SynthRAD2023 Grand Challenge dataset: Generating synthetic CT for radiotherapy. *Medical Physics*, 2023, vol. 50 (7), 4664-4674, https://doi.org/10.1002/mp.16529
- **HUIJBEN E** ; **TERPSTRA ML** ; **GALAPON JR A** ; **PAI S** ; **THUMMERER A** ; **KOOPMANS P** ; **MASPERO M**. Generating Synthetic Computed Tomography for Radiotherapy: SynthRAD2023 Challenge Report. *arXiv:2403.08447*, 2024, https://doi.org/10.48550/arXiv.2403.08447
- **GOODFELLOW, I.** *NIPS 2016 Tutorial: Generative Adversarial Networks*, 2017, http://arxiv.org/abs/1701.00160
- **MAO, X.** ; **LI, Q.** ; **XIE, H.** ; **LAU, R.Y.K.** ; **WANG, Z.** ; **SMOLLEY, S.P.** *Least Squares Generative Adversarial Networks*, 2017, http://arxiv.org/abs/1611.04076
- **LIM, J.H.** ; **YE, J.C.** *Geometric GAN*, 2017, http://arxiv.org/abs/1705.02894
- **ZHANG, Z.** ; **LIU, Q.** ; **WANG, Y.** Road Extraction by Deep Residual U-Net. *IEEE Geosci. Re-mote Sensing Lett.*, 2018, vol. 15, 749-753, https://doi.org/10.1109/LGRS.2018.2802944
- **DAS IJ** ; **CHENG CW** ; **CAO M** ; **JOHNSTONE PA**. Computed tomography imaging parameters for inhomogeneity correction in radiation treatment planning. *J Med Phys.*, January 2016, vol. 41 (1), 3-11